(19) Europäisches Patentamt / European Patent Office / Office européen des brevets

(11) **EP 4 773 074 A1**

(12) **EUROPEAN PATENT APPLICATION**
published in accordance with Art. 153(4) EPC

(43) Date of publication:
**08.07.2026 Bulletin 2026/28**

(21) Application number: **24860007.4**

(22) Date of filing: **30.08.2024**

(51) International Patent Classification (IPC):
**G06T 7/00** *(2017.01)*      **G16C 10/00** *(2019.01)*
**G16C 20/40** *(2019.01)*

(52) Cooperative Patent Classification (CPC):
**G06T 7/00; G16C 10/00; G16C 20/40**

(86) International application number:
**PCT/JP2024/031415**

(87) International publication number:
**WO 2025/047982 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.08.2023 JP 2023141079**

(71) Applicant: **RIKEN**
**Wako-shi, Saitama 351-0198 (JP)**

(72) Inventor: **TOKUHISA Atsushi**
**Wako-shi, Saitama 351-0198 (JP)**

(74) Representative: **Buzzi, Notaro & Antonielli d'Oulx
S.p.A.
Corso Vittorio Emanuele II, 6
10123 Torino (IT)**

(54) **DEVICE FOR ESTIMATING ATOMIC STRUCTURE, METHOD FOR ESTIMATING ATOMIC STRUCTURE, AND PROGRAM FOR ESTIMATING ATOMIC STRUCTURE**

(57)   (Solving Means) There is provided an atomic structure estimation apparatus for a biomolecule, including a generation unit which generates a generation image based on a target biomolecule; an acquisition unit which acquires, from a plurality of prepared atomic structures, a plurality of first candidate structure images based on two-dimensional projection images; a global search unit which selects, as a basic candidate structure, an atomic structure that has a high degree of similarity to the target biomolecule in structure and/or molecular orientation, from among a plurality of candidate structures of atomic structures, based on a degree of similarity between the generation image and a candidate structure image which is included in the plurality of first candidate structure images; and a local search unit which acquires a plurality of second candidate structure images based on two-dimensional projection images of a plurality of modified atomic structures obtained by modifying the basic candidate structure, and selects an atomic structure that has a high degree of similarity to the target biomolecule in structure and/or molecular orientation, from among the modified atomic structures, based on a degree of similarity between a second candidate structure image which is included in the plurality of second candidate structure images and the generation image.

FIG.1

**Description**

TECHNICAL FIELD

The present invention relates to an atomic structure estimation apparatus, an atomic structure estimation method, and an atomic structure estimation program.

BACKGROUND ART

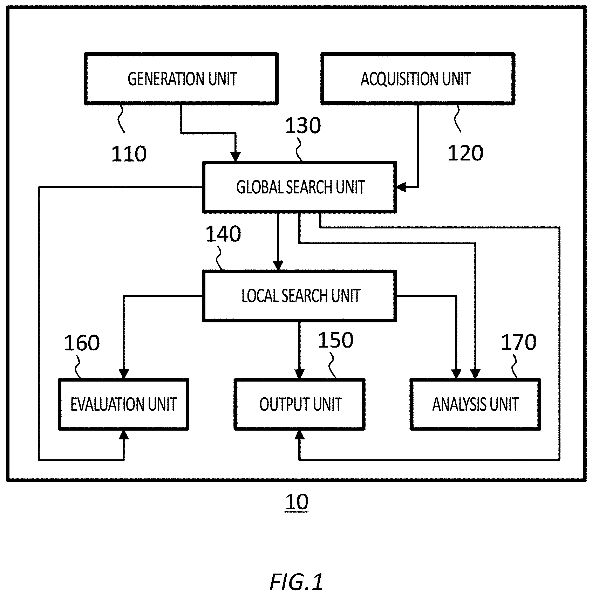

A method for searching an atomic structure of a large molecule such as a biomolecule is needed. The biomolecule is highly flexible, and it might be difficult to elucidate its diverse structures and state transitions solely from an observation image. Non-Patent Document 1 discloses that "As a new method for determining the structure of proteins, single particle structure analysis by cryo-electron microscopy has attracted attention. This method has less restrictions on samples, thus covering specimens with a wide molecular mass from small protein complexes of tens of kilo Daltons to large virus particles of several hundred mega Daltons in the same procedure (Figure 1). In addition, the spatial degree of freedom makes it possible to analyze dynamic structural changes and kinetics of proteins"(summary). Non-Patent Document 2 discloses that "Using cryoDRGN, we uncovered residual heterogeneity in high-resolution datasets of the 80S ribosome and the RAG complex, revealed a new structural state of the assembling 50S ribosome, and visualized large-scale continuous motions of a spliceosome complex. CryoDRGN contains interactive tools to visualize a dataset's distribution of per-particle variability, generate density maps for exploratory analysis, extract particle subsets for use with other tools and generate trajectories to visualize molecular motions"(summary). Non-Patent Document 3 discloses that "Cryo-electron microscopy (cryo-EM) is a powerful technique for determining the structure of proteins and other macromolecular complexes at near-atomic resolution. In single particle cryo-EM, the central problem is to reconstruct the three-dimensional structure of a macromolecule from 104-7 noisy and randomly oriented two-dimensional projections. However, the imaged protein complexes may exhibit structural variability, which complicates reconstruction and is typically addressed using discrete clustering approaches that fail to capture the full range of protein dynamics. Here, we introduce a novel method for cryo-EM reconstruction that extends naturally to modeling continuous generative factors of structural heterogeneity. This method encodes structures in Fourier space using coordinate-based deep neural networks, and trains these networks from unlabeled 2D cryo-EM images by combining exact inference over image orientation with variational inference for structural heterogeneity. We demonstrate that the proposed method, termed cryoDRGN, can perform ab initio reconstruction of 3D protein complexes from simulated and real 2D cryo-EM image data. To our knowledge, cryoDRGN is the first neural network-based approach for cryo-EM reconstruction and the first end-to-end method for directly reconstructing continuous ensembles of protein structures from cryo-EM images"(summary). Non-Patent Document 4 discloses that "Modeling flexible macromolecules is one of the foremost challenges in single-particle cryogenic-electron microscopy (cryo-EM), with the potential to illuminate fundamental questions in structural biology. We introduce Three-Dimensional Flexible Refinement (3DFlex), a motion-based neural network model for continuous molecular heterogeneity for cryo-EM data. 3DFlex exploits knowledge that conformational variability of a protein is often the result of physical processes that transport density over space and tend to preserve local geometry. From two-dimensional image data, 3DFlex enables the determination of high-resolution 3D density, and provides an explicit model of a flexible protein's motion over its conformational landscape. Experimentally, for large molecular machines (tri-snRNP spliceosome complex, translocating ribosome) and small flexible proteins (TRPV1 ion channel, $\alpha V\beta 8$ integrin, SARS-CoV-2 spike), 3DFlex learns nonrigid molecular motions while resolving details of moving secondary structure elements. 3DFlex can improve 3D density resolution beyond the limits of existing methods because particle images contribute coherent signal over the conformational landscape" (summary).

RELATED ART DOCUMENTS

NON-PATENT DOCUMENT

Non-Patent Document 1: Chihong SONG and Kazuyoshi MURATA, "Cryo-electron Microscopy for Structural Analysis of Dynamic Biological Macromolecules", J. Comput. Chem. Jpn., Vol. 17, No. 1, pp. 38-45 (2018)
Non-Patent Document 2: Ellen D. Zhong, Tristan Bepler, Bonnie Berger, Joseph H. Davis, "CryoDRGN: reconstruction of heterogeneous cryo-EM structures using neural networks", Nature Methods, volume 18, pages 176-185 (2021), https://doi.org/10.1038/s41592-020-01049-4
Non-Patent Document 3: Ellen D. Zhong, Tristan Bepler, Joseph H. Davis, Bonnie Berger, "Reconstructing continuous distributions of 3D protein structure from cryo-EM images", https://arxiv.org/abs/1909.05215, [Submitted on 11 Sep 2019 (v1), last revised 15 Feb 2020 (this version, v3)]

Non-Patent Document 4: Ali Punjani, David J. Fleet, "3DFlex: determining structure and motion of flexible proteins from cryo-EM", Nature Methods, volume 20, pages 860-870 (2023), https://www.nature.com/articles/s41592-023-01853-8

GENERAL DISCLOSURE

In a first aspect of the present invention, there is provided an atomic structure estimation apparatus for a biomolecule. The atomic structure estimation apparatus includes a generation unit, an acquisition unit, a global search unit, and a local search unit. The generation unit generates a generation image based on a target biomolecule. The acquisition unit acquires, from a plurality of prepared atomic structures, a plurality of first candidate structure images based on two-dimensional projection images. The global search unit selects, as a basic candidate structure, an atomic structure that has a high degree of similarity to the target biomolecule in structure and/or molecular orientation, from among a plurality of atomic structures, based on a degree of similarity between the generation image and a first candidate structure image which is included in the plurality of first candidate structure images. The local search unit acquires a plurality of second candidate structure images based on two-dimensional projection images of a plurality of modified atomic structures obtained by modifying the basic candidate structure, and selects an atomic structure that has a high degree of similarity to the target biomolecule in structure and/or molecular orientation, from among the modified atomic structures, based on a degree of similarity between a second candidate structure image which is included in the plurality of second candidate structure images and the generation image.

In the above description, the acquisition unit may acquire the plurality of first candidate structure images, based on two-dimensional projection images projected from a plurality of orientations surrounding an atomic structure which is included in the plurality of prepared atomic structures.

In the above description, the acquisition unit may acquire the first candidate structure image, based on a two-dimensional projection image of an atomic structure which is included in the plurality of prepared atomic structures and which is registered in a molecular structure database.

In the above description, the acquisition unit may acquire the first candidate structure image, based on a two-dimensional projection image of an atomic structure which is included in the plurality of prepared atomic structures and which is predicted from an amino acid sequence.

In the above description, the acquisition unit may acquire the first candidate structure image, based on a two-dimensional projection image of an atomic structure which is included in the plurality of prepared atomic structures and which is reproduced by a molecular dynamics simulation.

In the above description, the local search unit may increase a degree of similarity to the target biomolecule, by repeatedly modifying the basic candidate structure a plurality of times.

In the above description, the local search unit may simulate a structural transition of a modified atomic structure which is included in the plurality of modified atomic structures and which is selected, by a cascade selection-based molecular dynamics simulation (PaCS-MD).

In the above description, the global search unit may match the plurality of first candidate structure images with each of a plurality of generation images which is the generation image, and select the basic candidate structure based on a degree of similarity that is a matching result.

In the above description, the global search unit may select the basic candidate structure based on a highest value, a lowest value, an average value, and/or a median value of degrees of similarity of the plurality of first candidate structure images matched with each of the plurality of generation images.

In the above description, the global search unit may compute the degree of similarity between the generation image and the first candidate structure image by a parallel computation.

In the above description, the generation unit may generate the generation image based on a particle image of the target biomolecule. The generation unit may reduce noise in the particle image. The generation image may be a particle image in which the noise is reduced.

In the above description, the generation unit may reduce the noise in the particle image by an image generation by machine learning.

In the above description, the generation unit may apply a Fourier transform to the particle image in which the noise is reduced, and generates, as the generation image, a target wavenumber-space image that is an image after the Fourier transform. The acquisition unit may apply the Fourier transform to a two-dimensional projection image of an atomic structure, and acquire, as the first candidate structure image, a wavenumber-space image that is the image after the Fourier transform.

In the above description, the generation unit may exclude a set of irrelevant images in a latent space.

In the above description, the structure estimation apparatus may further include an output unit which outputs a three-dimensional structure model of a modified atomic structure which is included in the plurality of modified atomic structures and which is selected by the local search unit.

In the above description, the structure estimation apparatus may further include an evaluation unit which evaluates and outputs interactions between domains of a modified atomic structure which is included in the plurality of modified atomic structures and which is selected by the local search unit.

In the above description, the structure estimation apparatus may further include an analysis unit which analyzes structural features of the basic candidate structure and/or the modified atomic structure selected by the local search unit, by a principal component analysis. The output unit may plot and output results of the principal component analysis, on a plane or in a space.

In the above description, the global search unit may attribute, based on the degree of similarity, the generation image to any of basic candidate structures, each of which is the basic candidate structure. The output unit may further display, on the plane or in the space, a number of at least one attribution of the generation image for each of the basic candidate structures.

In the above description, the output unit may display the number of attributions, as a heatmap, on the plane or in the space.

In the above description, the output unit may display a structural transition of the target biomolecule, as a video, by continuously displaying the generation image attributed to the basic candidate structure which is adjacent, on the plane or in the space.

In a second aspect of the present invention, there is provided an atomic structure estimation method. The atomic structure estimation method includes generating, acquiring, performing a global search, and performing a local search. The generating may be generating a generation image based on a target biomolecule. The acquiring may be acquiring, from a plurality of prepared atomic structures, a plurality of first candidate structure images based on two-dimensional projection images. The performing a global search may be selecting, as a basic candidate structure, an atomic structure that has a high degree of similarity to the target biomolecule in structure and/or molecular orientation, from among a plurality of atomic structures, based on a degree of similarity between the generation image and a candidate structure image which is included in the plurality of first candidate structure images. The performing a local search may be acquiring a plurality of second candidate structure images based on two-dimensional projection images of a plurality of modified atomic structures obtained by modifying the basic candidate structure, and selecting an atomic structure that has a high degree of similarity to the target biomolecule in structure and/or molecular orientation, from among the modified atomic structures, based on a degree of similarity between a second candidate structure image which is included in the plurality of second candidate structure images and the generation image.

In a third aspect of the present invention, there is provided a program. The program is executed by a computer, and causes the computer to function as a generation unit, an acquisition unit, a global search unit, and a local search unit. The generation unit generates a generation image based on a target biomolecule. The acquisition unit acquires, from a plurality of prepared atomic structures, a plurality of first candidate structure images based on two-dimensional projection images. The global search unit selects, as a basic candidate structure, an atomic structure that has a high degree of similarity to the target biomolecule in structure and/or molecular orientation, from among a plurality of atomic structures, based on a degree of similarity between the generation image and a first candidate structure image which is included in the plurality of first candidate structure images. The local search unit acquires a plurality of second candidate structure images based on two-dimensional projection images of a plurality of modified atomic structures obtained by modifying the basic candidate structure, and selects an atomic structure that has a high degree of similarity to the target biomolecule in structure and/or molecular orientation, from among the modified atomic structures, based on a degree of similarity between a second candidate structure image which is included in the plurality of second candidate structure images and the generation image.

Note that the summary clause does not necessarily describe all necessary features of the embodiments of the present invention. The present invention may also be a sub-combination of the features described above.

BRIEF DESCRIPTION OF THE DRAWINGS

Fig. 1 shows the configuration of an atomic structure estimation apparatus 10 according to the present embodiment.

Fig. 2 shows an atomic structure estimation flow by the atomic structure estimation apparatus 10 according to the present embodiment.

Fig. 3 shows examples of particle images before and after a VAE is applied.

Fig. 4 shows an acquisition example of a two-dimensional projection image by an acquisition unit 120.

Fig. 5 shows an acquisition example of a two-dimensional projection image by an acquisition unit 120.

Fig. 6 shows an example of a selection of a basic candidate structure by a global search unit 130.

Fig. 7 shows an example of a result of a global structure search.

Fig. 8 shows an example of a result of a local structure search.

Fig. 9 shows an example of a three-dimensional structure model of a selected atomic structure according to the present embodiment.

Fig. 10 shows an example in which the number of attributions of generation images is indicated as a heatmap.

Fig. 11A shows an example of a state transition of the basic candidate structure.

Fig. 11B shows an example of a three-dimensional density map for each particle image set attributed to a plurality of representative structures.

Fig. 12 shows an example of a video display by an output unit 150.

Fig. 13 shows an example of a video display by the output unit 150.

Fig. 14 shows an example of a structural space and the structure.

Fig. 15 shows an example of a search result for Target 1.

Fig. 16 shows an example of a search result for a representative cluster.

Fig. 17 shows an example of a structure estimated to be a wild type.

Fig. 18 shows an example of a structure estimated to be a mutant type.

Fig. 19 shows an example of a computer 2200 in which a plurality of aspects of the present invention may be embodied entirely or partially.

DESCRIPTION OF EXEMPLARY EMBODIMENTS

The present invention will be described below through embodiments of the invention, but the following embodiments do not limit the invention according to the claims. Further, not all of combinations of features described in the embodiments are essential to the solving means of the invention.

Fig. 1 shows the configuration of an atomic structure estimation apparatus 10 according to the present embodiment. The atomic structure estimation apparatus 10 estimates an atomic structure of a biomolecule targeted for a structure analysis (hereinafter also referred to as a "target biomolecule"), based on a two-dimensional projection image of a prepared atomic structure and a particle image. The atomic structure estimation apparatus 10 includes a generation unit 110, an acquisition unit 120, a global search unit 130, a local search unit 140, an output unit 150, an evaluation unit 160, and an analysis unit 170.

The generation unit 110 generates a generation image based on the target biomolecule. For example, the generation unit 110 may generate the generation image based on a particle image of the target biomolecule. The generation image may be a particle image obtained by observing the target biomolecule with a cryo-electron microscope (CryoEM).

The generation image may be an image obtained by processing the particle image. For example, the generation unit 110 may reduce noise in the particle image, and set the particle image in which the noise is reduced as the generation image. The generation image may also be an image obtained by further performing the Fourier transform.

The acquisition unit 120 prepares the atomic structure in advance as a virtual molecule. For example, the acquisition unit 120 may acquire a structure recorded in a molecular structure database, a structure predicted from an amino acid sequence, and/or a structure reproduced by molecular dynamics simulation, and use it as the atomic structure for a seed model. Instead of or in addition to this, the acquisition unit 120 may further augment the atomic structure, by simulating a structural change of the atomic structure by a molecular dynamics simulation or a structure sampling engine.

The acquisition unit 120 acquires a plurality of two-dimensional projection images for the plurality of atomic structures. The acquisition unit 120 acquires a first candidate structure image based on the two-dimensional projection image.

The global search unit 130 globally searches an approximate atomic structure of the target biomolecule, and sets the searched atomic structure as a basic candidate structure. For example, first, the global search unit 130 acquires the generation image from the generation unit 110, and the first candidate structure image from the acquisition unit 120. From among the plurality of prepared atomic structures, the global search unit 130 selects, as the basic candidate structure, an atomic structure that has a high degree of similarity to the target biomolecule in structure and/or molecular orientation, based on a degree of similarity between the generation image and the first candidate structure image.

The global search unit 130 may be implemented by a parallel computation. For example, the global search unit 130 may be implemented by a supercomputer having a large number of computational nodes.

The local search unit 140 searches for a local atomic structure, with higher precision, based on the basic candidate structure. The local search unit 140 may modify the basic candidate structure to acquire a plurality of modified atomic structures. For example, the local search unit 140 may modify the basic candidate structure while maintaining the biomolecular structure to acquire the plurality of modified atomic structures. The local search unit 140 may continuously modify the basic candidate structure while maintaining the biomolecular structure to acquire the plurality of modified atomic structures. For example, the local search unit 140 performs a selection-based molecular dynamics simulation based on the basic candidate structure to acquire the plurality of modified atomic structures.

The local search unit 140 acquires a plurality of second candidate structure images based on the two-dimensional projection images of the plurality of modified atomic structures. A second candidate structure image may be an image obtained by processing the two-dimensional projection image. For example, the local search unit 140 may perform the Fourier transform on the two-dimensional projection image, and set it as the second candidate structure image.

The local search unit 140 selects an atomic structure that has a high degree of similarity to the target biomolecule in at least one of structure or molecular orientation, from among the modified atomic structures, based on the degree of similarity between the second candidate structure image and the generation image. For example, the local search unit 140 may select, from among the modified atomic structures, an atomic structure that has a high degree of similarity to the target biomolecule in both structure and molecular orientation. The local search unit 140 may be implemented by the parallel computation. For example, the local search unit 140 may be implemented by a supercomputer having a large number of computational nodes.

The output unit 150 outputs information relating to the basic candidate structure selected by the global search unit 130 and/or information relating to the modified atomic structure selected by the local search unit 140. For example, the output unit 150 outputs a three-dimensional structure model of the modified atomic structure.

The evaluation unit 160 evaluates a chemical property and/or a physical property of the basic candidate structure and/or the modified atomic structure. For example, the evaluation unit 160 evaluates and outputs interactions between fragments (residues) and/or between domains of the selected atomic structure among the modified atomic structures.

The analysis unit 170 analyzes structural features of the basic candidate structure and/or the modified atomic structure. For example, the analysis unit 170 analyzes a structural feature of the modified atomic structure by a principal component analysis.

In this way, with the atomic structure estimation apparatus 10 according to the present embodiment, matching is performed based on the projection image of the atomic structure reproduced by the molecular dynamics simulation and the particle image, and a search is performed according to the result thereof. With the present embodiment, by performing a comprehensive analysis using single noisy particle images (for example, the CryoEM particle images) based on the target biomolecule, it is possible to realize a single image analysis that directly estimates the diverse three-dimensional atomic structures of the biomolecule.

With the present embodiment, in comparison with a method such as a multi-image analysis that enhances a resolution of a common structural portion by averaging a plurality of CryoEM particle images and removing the noise, it is possible to specify the three-dimensional atomic structure at a higher resolution without losing diverse structures due to averaging.

With a method which reproduces the three-dimensional structure based on the image obtained by averaging the particle images, the structural feature of the target biomolecule, in a flexible region, is lost due to averaging. On the other hand, with the method of the present embodiment, individual generation image is linked to individual atomic structure reproduced by the molecular simulation, and thus the structural information of the biomolecule is not lost due to averaging, and it is possible to estimate the structure of the target biomolecule more precisely. In particular, with the present embodiment, it is possible to specify with higher precision the structure of a highly flexible region in the biomolecule (for example, a region where the structural information is easily lost due to averaging).

Further, with the present embodiment, by using both of the global search and the local search together, it is possible to efficiently search for the diverse structures of the target biomolecule with limited computational resources, and without sacrificing the precision. In addition, with the present embodiment, it is possible to output the three-dimensional structure of the target biomolecule.

The atomic structure estimation apparatus 10 may be implemented by a single apparatus or by a plurality of apparatuses (computers) with divided roles. When implemented by a plurality of apparatuses, the atomic structure estimation apparatus 10 may be a server/client system, and in this case, at least a part of functions of the atomic structure estimation apparatus 10 may be implemented by a server apparatus, and the remaining functions may be implemented by a client apparatus. The client apparatus may have only a function of transmitting an input from a user to the server apparatus and displaying an output from the server apparatus, and, in this case, a substantial function of the atomic structure estimation apparatus 10 is implemented solely by the server apparatus.

At least a part of the atomic structure estimation apparatus 10 may be implemented by a computing apparatus such as a supercomputer. The apparatus (computer) which is used for at least a part of the atomic structure estimation apparatus 10 may be a computer such as a PC (personal computer), a tablet computer, a smartphone, a workstation, a server computer, a cloud server, or a general-purpose computer, or may be a computer system in which a plurality of computers are connected.

Instead of this, the apparatus (computer) which is used for the atomic structure estimation apparatus 10 may be a dedicated computer designed according to each purpose, or may be dedicated hardware implemented by a dedicated circuit. In the atomic structure estimation apparatus 10, although not particularly described below, a memory, a hard disk, or the like is included, and information necessary for processing is appropriately stored, and the atomic structure estimation apparatus 10 may transmit the information between respective processing modules such as the generation unit 110 and the acquisition unit 120.

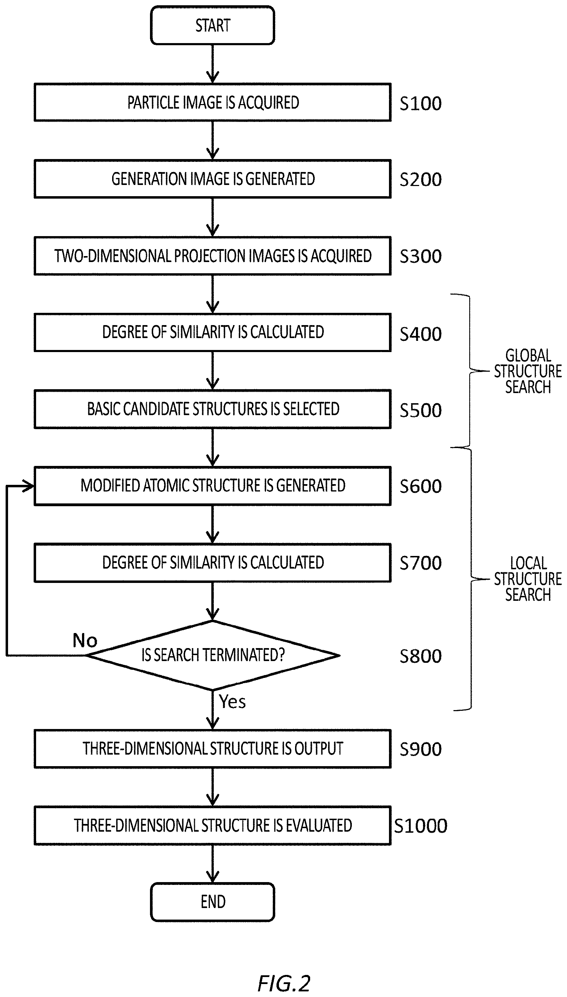

Fig. 2 shows an atomic structure estimation flow by the atomic structure estimation apparatus 10 according to the present embodiment. The atomic structure estimation apparatus 10 estimates the atomic structure of the target biomolecule by executing S100 to S1000. Some of S100 to S1000 may be omitted. In addition to S100 to S1000, another operation may be executed.

S100 to S900 may be an example of the single image analysis for estimating the three-dimensional atomic structure from the particle image. S1000 may be an example of the multi-image analysis for analyzing the diverse structural states based on the result of the single image analysis, and an example of a video presenting a motion of the target biomolecule.

In S100, the generation unit 110 acquires the particle image. The generation unit 110 acquires an image of the target biomolecule captured through observation by a particle analysis method using a transmission electron microscope. For example, the generation unit 110 acquires the image of the target biomolecule captured by the cryo-electron microscope (CryoEM).

The generation unit 110 acquires a plurality of particle images for the target biomolecule. In this manner, the generation unit 110 obtains the images of the target biomolecule captured from various orientations. The particle image may refer to a so-called single particle image; however, the target biomolecule serving as the target is not limited to a single particle. The target biomolecule may be the single particle, or a complex in which a plurality of single particle structures are aggregated, coordinated, or bonded.

For example, the target biomolecule may be a spike protein alone (a trimer consisting of the same three subunits), or a complex in which spike protein is bonded to an antibody (consisting of the trimer plus a number of antibodies bonded thereto). Below, an example of a case in which a wild type and a mutant type (D614G) of the spike protein derived from SARS-CoV-2 are set as the target biomolecule, will be described.

The generation unit 110 may exclude some of the plurality of particle images. For example, the generation unit 110 may perform multistage two-dimentional classification on the plurality of particle images, and exclude, based on class average images, particle images having a low probability of containing the biomolecule. As an example, the generation unit 110 may exclude particle images that were not classified into any class.

Next, in S200, the generation unit 110 generates the generation image from the particle image. For example, the generation unit 110 may reduce the noise in the particle image. For example, the generation unit 110 may reduce the noise in the particle image by the image generation by machine learning such as deep learning.

As an example, the generation unit 110 may input the particle image to a Variational Autoencoder (VAE), and acquire the image that is output as the generation image. The VAE is a combination of an encoder and a decoder, and it is possible to compress the particle image to dimensionality of a latent space by encoding the particle image with the encoder. Thereafter, it is possible to restore the image of the original size by decoding the compressed data with the decoder.

In the manifold hypothesis, the diverse structures that the biomolecule such as protein can adopt in a high-dimensional space can be regarded as a lower-dimensional manifold called the latent space. By passing through encoding and decoding by the VAE, it is possible to reduce the noise while preserving an essential feature of the particle image.

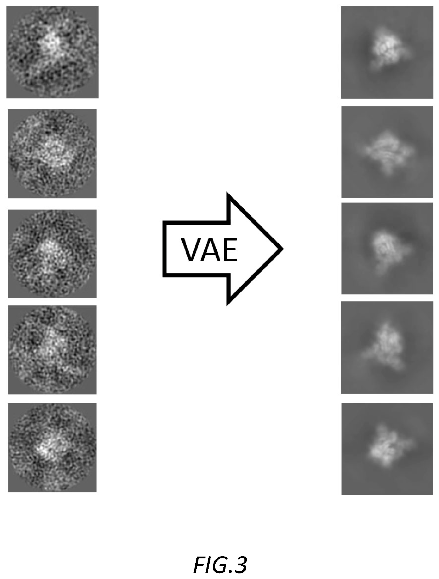

Fig. 3 shows examples of particle images before and after a VAE is applied. By applying the VAE to the image in the left side column of Fig. 3, it is possible to obtain the image in the right side column in which the noise is reduced.

In the above description, the generation unit 110 may apply a Fourier transform to the particle image with reduced noise, and generate a target wavenumber-space image resulting from the Fourier transform. For example, the generation unit 110 may apply a Fourier transform to an image output from the VAE in response to input of the particle image. The generation unit 110 may output the target wavenumber-space image as a generated image.

Instead of this, the generation unit 110 may output, as the generation image, the image obtained by inputting the particle image to the VAE to be output without applying the Fourier transform. Instead of this, the generation unit 110 may output the particle image itself as the generation image without executing the Fourier transform and the noise reduction.

The generation unit 110 may use all of the particle images as they are in which the noise is reduced, or instead of this, may select and use only a representative image. For example, the generation unit 110 may execute a clustering analysis based on an image distribution in the latent space when the particle image is input to the VAE, and classify a large number of particle images into clusters. An existing method such as X-means may be used as the clustering analysis.

The generation unit 110 may select a center image of each cluster as the representative image of the cluster, and use only the representative image as the particle image in subsequent processing. This makes it possible to reduce an amount of processing for a degree of similarity calculation, which will be described below.

The generation unit 110 may execute processing of excluding some clusters in the latent space as a set of irrelevant images. For example, the generation unit 110 may exclude, from the subsequent processing target, a cluster of which occupancy in the latent space is greater than or equal to a threshold value, and/or a cluster of which the representative image is unclear. The generation unit 110 may automatically determine the cluster to be excluded, or may exclude the cluster specified by the user.

In S300, the acquisition unit 120 acquires the plurality of two-dimensional projection images of the atomic structure of the virtual molecule. The acquisition unit 120 acquires a plurality of atomic structures of a known biomolecule (hereinafter also referred to as the "virtual molecule") from an existing molecular structure database. Examples of the molecular structure database include, for example, the Protein Data Bank (PDB), the Electron Microscopy Data Bank (EMDB), and/or the AlphaFold Protein Structure Database (PSD), or the like.

The acquisition unit 120 may use the structure predicted from the amino acid sequence. The acquisition unit 120 acquires the two-dimensional projection image of the atomic structure predicted from the amino acid sequence. The acquisition unit 120 may acquire the two-dimensional projection images projected from a plurality of orientations surrounding the atomic structure. The plurality of orientations may be predetermined orientations, and, for example, may be orientations that are separated at equal angular intervals.

The acquisition unit 120 may simulate a motion of the virtual molecule, the atomic structure of which is acquired, by using various simulation methods (for example, the molecular dynamics simulation or an OFLOOD method), and augment the structure. The acquisition unit 120 acquires the two-dimensional projection image of the atomic structure reproduced by the molecular dynamics simulation. The acquisition unit 120 may acquire the two-dimensional projection images projected from a plurality of orientations surrounding the atomic structure. The plurality of orientations may be predetermined orientations, and, for example, may be orientations that are separated at equal angular intervals.

The acquisition unit 120 may acquire a plurality of first candidate structure images based on the plurality of two-dimensional projection images. For example, when the generation image is a wavenumber-space image obtained by performing the Fourier transform, the acquisition unit 120 may apply the Fourier transform to the two-dimensional projection image of the atomic structure, and acquire, as the first candidate structure image, a wavenumber-space image that is the image after the Fourier transform. When the Fourier transform is not performed on the generation image, the acquisition unit 120 may set, as the first candidate structure image, the two-dimensional projection image as it is.

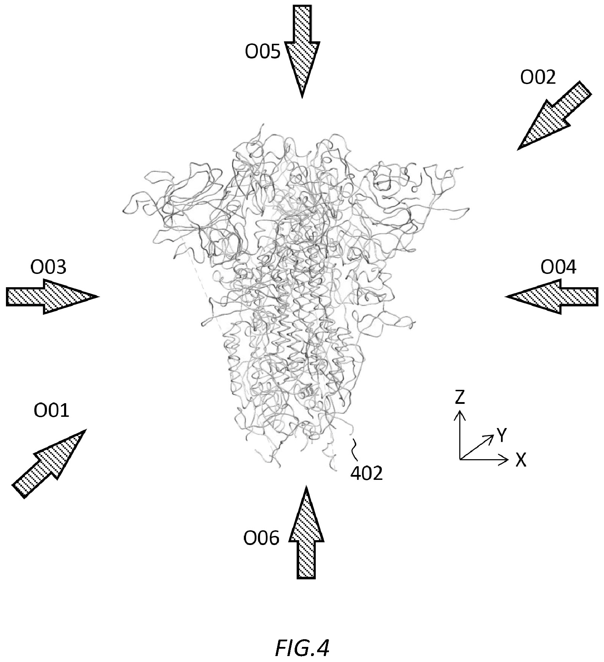

Fig. 4 shows an acquisition example of a two-dimensional projection image by the acquisition unit 120. As shown in Fig. 4, the acquisition unit 120 may acquire the images in which a virtual molecule 402 is two-dimensionally projected from a plurality of orientations (for example, O01 to O06). In Fig. 4, six orientations are shown for convenience of description; however, the acquisition unit 120 may acquire two-dimensional projection images from a very large number of orientations (for example, 10,000 orientations).

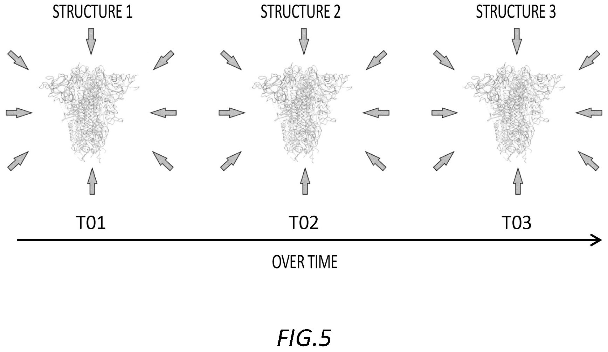

Fig. 5 shows an acquisition example of a two-dimensional projection image by the acquisition unit 120. As shown in Fig. 5, the acquisition unit 120 simulates the motion of the virtual molecule 402 over time by the molecular dynamics simulation, and acquires the two-dimensional projection image of the virtual molecule 402 at each of a plurality of time points. For example, in Fig. 5, the acquisition unit 120 acquires the two-dimensional projection images from the plurality of orientations shown in Fig. 4 at a time point T01, a time point T02, a time point T03, and so on.

Here, the two-dimensional projection images acquired at the same time point may be treated as being attributed to the same atomic structure. On the other hand, the atomic structure changes over time, and thus the structures become different from each other at different time points. For example, the atomic structure becomes a structure 1 at the time point T01, becomes a structure 2 at the time point T02, and becomes a structure 3 at the time point T03. The structures 1 to 3 are structures different from each other.

For example, when 100 virtual molecules, 100 orientations, and 100 time points are set, the acquisition unit 120 acquires $100 \times 100 \times 100 = 1,000,000$ two-dimensional projection images.

When the target biomolecule is a complex, the virtual molecule can adopt various states depending on aggregated, coordinated, and bonded states of each particle. Therefore, the acquisition unit 120 may prepare the virtual molecule for each of the aggregated, coordinated, and bonded states, and may execute a simulation for each virtual molecule. For example, for a spike protein complex to which three antibodies are bonded (that is, consisting of the trimer plus three antibodies), the acquisition unit 120 may prepare each virtual molecule for a case in which no antibody is bonded, a case in which an antibody is bonded at only one site, a case in which antibodies are bonded at two sites, and a case in which antibodies are bonded at all three sites.

In S400 to S500, the global search unit 130 executes the global structure search to globally search the approximate atomic structure of the target biomolecule.

First, in S400, the global search unit 130 acquires the generation image obtained in S200, and the first candidate structure image obtained in S300. The global search unit 130 calculates the degree of similarity between the generation image and the first candidate structure image. The global search unit 130 may implement the calculation of the degree of similarity between the images, by existing template matching methods such as the correlation value: Cross-Correlation, the mean square error: Mean-Square-Error, the squared error: Squared-Difference, and the absolute value error: Absolute-Difference.

As an example, for wavenumber-space images i and j, the global search unit 130 may calculate, as a correlation value, $c_{ij}(\xi, \alpha)$ which is represented by Expressions 1 to 3. According to Expressions 1 to 3, the correlation value is calculated after an intensity image is normalized by wavenumber dependence of an expected value, and thus it is possible to capture a correlation over a wider wavenumber range and to utilize even a weaker signal as an effective signal.

(Math. 1)

$$c_{ij}(\xi, \alpha) = \frac{\Psi_{ij}(\xi, \alpha)}{\bar{s}_i(\xi)\bar{s}_j(\xi)} - 1$$

… Expression 1

(Math. 2)

$$\Psi_{ij}(\xi, \alpha) = \frac{1}{N_\xi} \sum_{l=0}^{N_\xi - 1} s_i\left(\xi, \frac{2\pi l}{N_\xi}\right) s_j\left(\xi, \frac{2\pi l}{N_\xi} + \alpha\right)$$

… Expression 2

(Math. 3)

$$\bar{s}_i(\xi) = \frac{1}{N_\xi} \sum_{l=0}^{N_\xi - 1} s_i\left(\xi, \frac{2\pi l}{N_\xi}\right)$$

… Expression 3.

Here, a calculation example of the degree of similarity using the correlation value $c_{ij}(\xi, \alpha)$ will be described. The global search unit 130 may calculate a degree of similarity $R_{c,ij}(k^{\wedge}_{ij}, \alpha^{\wedge}_{ij})$ by using Expressions 4 to 7. A rotation of the molecule with respect to an incident probe light axis appears as a rotation about the center in the wavenumber-space image.

In consideration of a rotational degree of freedom $\alpha$, it is possible to obtain, at once by a correlation function, the correlation value for a 360-degree rotation of a pattern with respect to the two-dimensional plane. A diagram in which the correlation value is displayed as a function of $\alpha$ and $k = (2/\lambda)\sin(\xi/2)$ is referred to as a correlation diagram. Here, $\xi = 2\theta$, which represents the scattering angle. In addition, s represents the wavenumber-space intensity of the observed image, s represents the expected value of the intensity count, $\lambda$ represents the wavelength of the incident probe light, and $N_\xi$ represents the number of discrete pixels on the concentric circle, respectively. Further, the integrated correlation value (or integrated correlation diagram) $I_{c,ij}(kup, \alpha)$ is defined by integrating the correlation value $c_{ij}(\xi, \alpha)$ in the $\alpha$ direction and the k direction. By performing this integration, it is possible to reduce the influence of noise.

$\lambda$ represents the wavenumber of the incident probe light. $k_c$ has a has a magnitude equal to the inverse of the molecular size as a correlation length of the wavenumber-space pattern. In addition, $k_{up}$ represents the maximum wavenumber of the wavenumber-space image used for the similarity determination calculation. In the integral correlation diagram, the direction of the correlation line is set as $\alpha^\wedge$, and the maximum value of the integrated correlation value along the k direction at $\alpha^\wedge$ is set as $I_{c,ij}(k^\wedge_{ij}, \alpha^\wedge_{ij})$. By normalizing with the autocorrelation term of each of the wavenumber-space images obtained in a similar manner, it is possible to quantify the degree of similarity $R_{c,ij}(k^\wedge_{ij}, \alpha^\wedge_{ij})$ which is a matching score obtained by normalizing the similarity of patterns of a pair of wavenumber-space images.

(Math. 4)

$$I_{c,ij}\left(k_{up}, \alpha\right) = \int_0^{\xi_{up}} i_{c,ij}(\xi, \alpha) \sin\xi d\xi$$

… Expression 4

(Math. 5)

$$= \lambda^2 \int_0^{k_{up}} i_{c,ij}(k, \alpha) k dk$$

… Expression 5

(Math. 6)

$$i_{c,ij}(k, \alpha) = \frac{2\pi}{N_\xi} \sum_{\Delta\alpha = -2k_c/k}^{2k_c/k} c_{ij}(\xi, \alpha + \Delta\alpha)$$

… Expression 6

(Math. 7)

$$R_{c,ij}(\hat{k}_{ij}, \hat{\alpha}_{ij}) = \frac{I_{c,ij}(\hat{k}_{ij}, \hat{\alpha}_{ij})}{\sqrt{I_{c,ii}(\hat{k}_{ii}, \hat{\alpha}_{ii})}\sqrt{I_{c,jj}(\hat{k}_{jj}, \hat{\alpha}_{jj})}}$$

... Expression 7.

The similarity between the target structure and the candidate structure is estimated via the wavenumber-space image; however, even for the same candidate structure, when the molecular orientation, which is the direction in which the probe light is incident, is different, the pattern of the wavenumber-space image is different. Therefore, in performing the search for a similar structure in the wavenumber-space, it is necessary to specify the molecular orientation similar to that of the target image (molecular orientation search). For each candidate structure, images with a plurality of different orientations are prepared, and the highest $R_c$ value obtained as a result of the molecular orientation search can be set as a structural matching degree of the candidate structure.

The global search unit 130 calculates the degree of similarity between each of a large number of generation images and each of the first candidate structure images. For example, in a case where N generation images and M first candidate structure images exist, the global search unit 130 may calculate the degree of similarity for all or some of the combinations ($N \times M$ possible combinations) of the generation images and the first candidate structure images. The global search unit 130 may compute the degree of similarity between the generation image and the first candidate structure image by the parallel computation.

In S500, the global search unit 130 selects, from the plurality of virtual molecules, the virtual molecule serving as the basic candidate structures. The global search unit 130 selects the basic candidate structure based on the degree of similarity calculated in the S400. The global search unit 130 may match a plurality of first candidate structure images with each of a plurality of generation images for each virtual molecule, and select the basic candidate structure based on the degree of similarity that is the matching result.

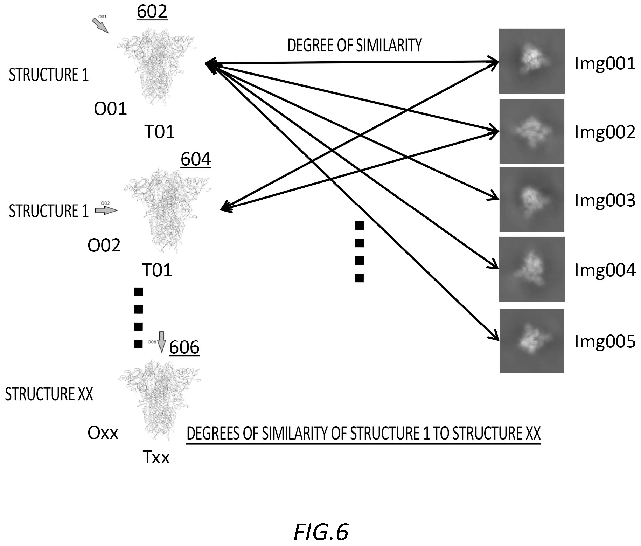

Fig. 6 shows an example of a selection of a basic candidate structure by the global search unit 130. For example, the global search unit 130 calculates the degree of similarity between a first candidate structure image 602 which is obtained by projecting the virtual molecule shown by the structure 1 from the orientation O01 at the time point T01 and the plurality of generation images (a generation image Img 001, a generation image Img 002, a generation image Img 003, a generation image Img 004, a generation image Img 005, a generation image Img 006, ...), respectively.

The global search unit 130 similarly calculates, with the plurality of generation images, the degrees of similarity of a first candidate structure image 604 for which the time point is the same as but the orientation is different from that of the first candidate structure image 602. The time points are the same, and thus the first candidate structure image 602 and the first candidate structure image 604 are both treated as being attributed to the same structure (the structure 1).

The global search unit 130 similarly calculates, with the plurality of generation images, the degrees of similarity of a first candidate structure image 606 at a time point TXX after time has elapsed from T01. Here, a transition to a structure XX occurs as the time elapses, the first candidate structure image 606 is treated as being attributed to the structure XX.

The global search unit 130 comprehensively determines these degrees of similarity, thereby calculating a structural fitness score which is a degree to which the virtual molecule shown by each structure (for example, the "structure 1") is similar to the target biomolecule in structure and molecular orientation. For example, among the degrees of similarity of all the orientations (O01, O02, ...) for the virtual molecule shown by the structure 1 (hereinafter also referred to as "all degrees of similarity of the structure 1"), the global search unit 130 may set the highest value as the structural fitness score between the structure 1 and the target biomolecule.

As the structural fitness score between the structure 1 and the target biomolecule, the global search unit 130 may set the lowest value, the average value, and/or the median value of a predetermined number of top-ranked values of all degrees of similarity of the structure 1, for the virtual molecule shown by the structure 1. As the structural fitness score between the structure 1 and the target biomolecule, the global search unit 130 may set the number related to the generation images, the degree of similarity of which exceeds the threshold value. For example, as the structural fitness score between the structure 1 and the target biomolecule, the global search unit 130 may set the number of the generation images, the degree of similarity of which exceeds the threshold value, or a sum of the degrees of similarity of the generation images, the degree of similarity of which exceeds the threshold value.

The global search unit 130 calculates the structural fitness scores for the atomic structures (for example, the structure 1, the structure 2, the structure 3, ...) of the plurality of virtual molecules. The global search unit 130 may select the basic candidate structure based on the structural fitness score.

For example, as the basic candidate structures, the global search unit 130 may select a predetermined number of atomic structures having top-ranked structural fitness scores among the plurality of atomic structures, and/or the atomic structures, the structural fitness score of which exceeds the threshold value. In this way, the global search unit 130 can select the basic candidate structure based on the highest value, the lowest value, the average value, and/or the median value of the degrees of similarity of the plurality of first candidate structure images matched with each of the plurality of generation images.

As described above, the first candidate structure image and the generation image can be wavenumber-space images. When the global search unit 130 calculates the degree of similarity in the wavenumber-space image, it is possible to evaluate the similarity of the structures in more consideration of translational and rotational degrees of freedom. When a plurality of basic candidate structures are selected, processing S600 and the subsequent processing which will be described below may be executed in parallel for each basic candidate structure.

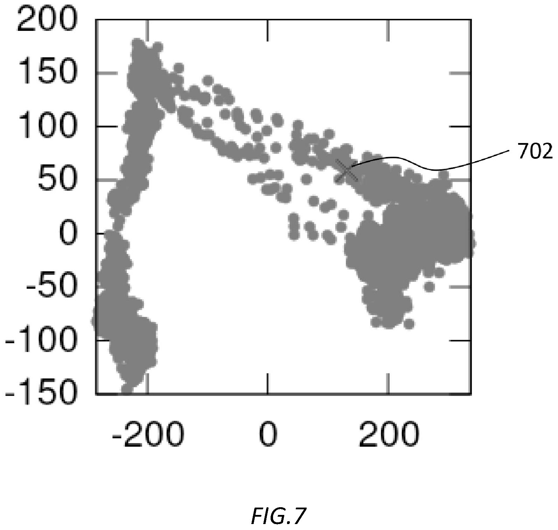

Fig. 7 shows an example of a result of a global structure search. The graph in the figure shows the result in which features of the atomic structures of the plurality of virtual molecules are plotted on two dimensions by the principal component analysis. The respective gray points in the graph correspond to the virtual molecules of the plurality of virtual molecules prepared in S300. The global search unit 130 may select a basic candidate structure 702 from among the plurality of virtual molecules.

In S600 to S700, the local search unit 140 executes a local structure search of the target biomolecule based on the basic candidate structure. In the local structure search, the local search unit 140 increases the degree of similarity to the target biomolecule, by repeatedly modifying the basic candidate structure a plurality of times. The local search unit 140 may continuously increase the degree of similarity to the target biomolecule. The local search unit 140 performs the local structure search while maintaining the biomolecular structure and generating a new structure on-the-fly.

In a case where there are a plurality of basic candidate structures, the processing of S600 to S700 may be performed for each basic candidate structure. In that case, the processing in parallel may be performed for each basic candidate structure.

In S600, the local search unit 140 may perform the selection-based molecular dynamics simulation by setting the basic candidate structure as a starting structure. A plurality of modified atomic structures obtained by modifying the basic candidate structure while maintaining the biomolecular structure based on the force field, are acquired. The biomolecular structure is a structure that the biomolecule can adopt chemically, physically, and/or biologically, and may be a structure that is defined by a rule relating to a structure that is input in advance. For example, the local search unit 140 may modify the structure while maintaining the biomolecular structure, by restricting bonded terms (bond lengths, bond angles, dihedral angles, and the like) and non-bonded terms (long-range electrostatic forces and van der Waals forces) of atoms constituting a molecule. In a specific example, the local search unit 140 may restrict, by the Ramachandran plot, the scope of angles that the dihedral angles can adopt between atoms or sets of atoms in the biomolecular structure such as protein.

The local search unit 140 may use, more generally, a force field parameter set that physically and chemically defines the ease with which the states of the biomolecular structure can be adopted, or an objective function defined separately. With energy values or score values obtained from such an objective function, as indices, the local search unit 140 may execute the modifications while restricting the structures that the biomolecule can adopt.

The modification of the basic candidate structure is performed by a structural transition from the basic candidate structure over time. As the selection-based molecular dynamics simulation, for example, the cascade selection-based molecular dynamics simulation (PaCS-MD) may be used. The local search unit 140 may simulate the structural transition of the modified atomic structure by the cascade selection-based molecular dynamics simulation (PaCS-MD).

A known algorithm may be used as the algorithm of the PaCS-MD. For example, a method which repeats the following (A) and (B) can be employed: (A) executing the molecular dynamics simulation (MD) at a constant temperature starting from the plurality of modified atomic structures; and (B) performing ranking based on the degree of similarity to the target structure (the degree of similarity calculated in S700 which will be described below), and restarting a plurality of short-time molecular dynamics simulations by setting, as new initial structures, a plurality of top-ranked structures (a predetermined number of top-ranked structures).

The local search unit 140 acquires a plurality of two-dimensional projection images of the simulated modified atomic structure. The local search unit 140 may acquire a large number of two-dimensional projection images of the modified atomic structure by a method similar to that of the acquisition unit 120 in S300. For example, for each of the modified atomic structures, the local search unit 140 may acquire the two-dimensional projection images from a plurality of orientations at a plurality of time points.

The local search unit 140 may acquire a plurality of second candidate structure images based on the two-dimensional projection images of the modified atomic structure. For example, when the generation image is a wavenumber-space image obtained by performing the Fourier transform, the local search unit 140 may apply the Fourier transform to the two-dimensional projection image of the modified atomic structure, and acquire, as the second candidate structure image, a wavenumber-space image that is an image after the Fourier transform. When the Fourier transform is not performed on the generation image, the local search unit 140 may set, as the second candidate structure image, the two-dimensional projection image as it is.

In S700, the local search unit 140 calculates the degree of similarity between the second candidate structure image and the generation image obtained in S200. The local search unit 140 may calculate the degree of similarity by a method similar to that of the global search unit 130 in S400.

For each modified atomic structure, the local search unit 140 may calculate the degree of similarity between the plurality of second candidate structure images and the generation image, and calculate the structural fitness score between the modified atomic structure and the target biomolecule based on the degree of similarity. The local search unit 140 may calculate the structural fitness score from the degree of similarity by a method similar to that of the global search unit 130 in S500.

In S800, the local search unit 140 determines whether to terminate the local structure search. When it is determined that a search termination condition is satisfied, the local search unit 140 may terminate the local structure search for the basic candidate structure for which the search is being executed. The search termination condition may be that "the structural fitness score of the modified atomic structure exceeds the threshold value", that "the degree of improvement in the structural fitness score of the modified atomic structure in the repetition of S600 to S800 becomes lower than or equal to the threshold value", that "S600 to S800 have been repeated a predetermined number of times", and/or that "a predetermined time has elapsed", or the like.

The local search unit 140 may terminate the repetition when the number of repetitions input by the user is reached.

When the local structure searches of all of the basic candidate structures are terminated, the local search unit 140 selects, as the modified atomic structure, an atomic structure having a high structural fitness score with respect to the target biomolecule, from among the modified atomic structures searched up to that time point. The modified atomic structure selected by the local search unit 140 is hereinafter also referred to as a "selected atomic structure". The local search unit 140 may select a plurality of modified atomic structures (for example, a predetermined number of modified atomic structures having the top-ranked structural fitness score), as the selected atomic structures.

When the local search unit 140 terminates the local structure search, the processing proceeds to S900. Otherwise, the local search unit 140 returns the processing to S600 and continues the local structure search. In the subsequent S600, the local search unit 140 may further modify the modified atomic structure having the top-ranked structural fitness score to set a new modified atomic structure, and continue the search. In this way, the local search unit 140 may repeat the modification of the atomic structure starting from the basic candidate structure, a plurality of number of times, thereby continuously increasing the structural fitness score.

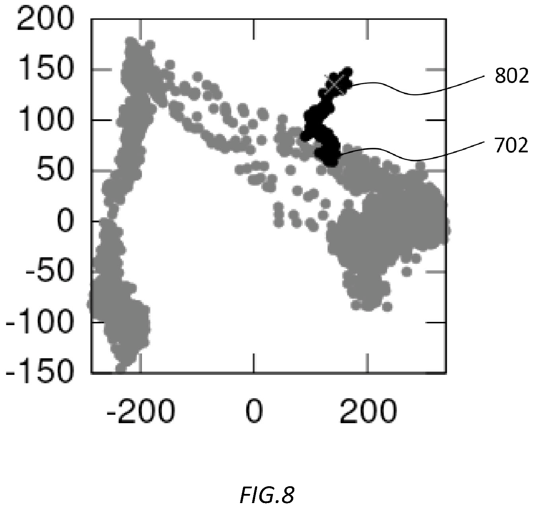

Fig. 8 shows an example of a result of a local structure search. The graph in the figure shows the result in which features of the atomic structures are plotted on two dimensions by the principal component analysis, similarly to Fig. 7. The black circle in the figure indicates the modified atomic structures that are generated from the basic candidate structure 702 by repeating the modification. The local search unit 140 finally selects the selected atomic structure 802.

Fig. 8 shows that, in the local structure search, the atomic structure is gradually modified, starting from the basic candidate structure 702. In this way, with the present embodiment, it is possible to search for a structure that is more suitable for the target biomolecule, by searching for the approximate structure close to the target biomolecule by the global structure search and then executing the local structure search.

In the global search and/or the local search, instead of performing the all-orientation (10, 240) search for each candidate structure, a small number of advantageous molecular orientations may be searched for by Bayesian optimization. This makes it possible to reduce a calculation cost.

In S900, the output unit 150 outputs the result obtained by the search. For example, the output unit 150 outputs the information relating to the basic candidate structure, the modified atomic structure, and/or the selected atomic structure. As an example, the output unit 150 outputs a three-dimensional structure model of the selected atomic structure.

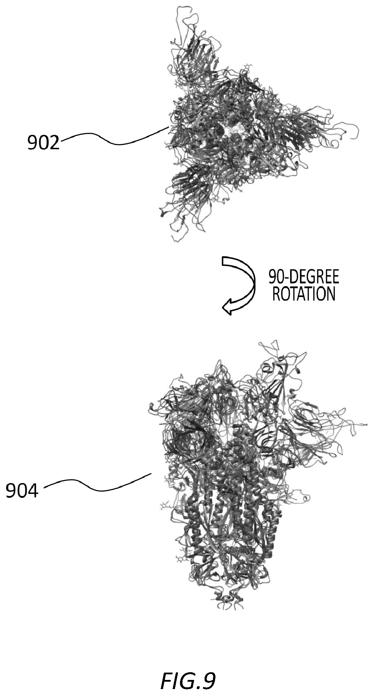

Fig. 9 shows an example of a three-dimensional structure model of a selected atomic structure according to the present embodiment. The output unit 150 may output a three-dimensional structure model 902 for the selected atomic structure, for displaying on an output screen. For the three-dimensional structure model 902, the output unit 150 may receive an operation from the user.

For example, the operations such as rotation, enlargement, reduction, horizontal movement, approach, and separation of the three-dimensional structure model 902, are input, and according to the operations, the output unit 150 may change the display of the three-dimensional structure model 902. As an example, according to the input of the operation of the 90-degree rotation by the user, the output unit 150 may output, on the output screen, a three-dimensional structure model 904 obtained by rotating the three-dimensional structure model 902 by 90 degrees.

The output unit 150 may output a result obtained by the analysis unit 170 analyzing the basic candidate structure, the modified atomic structure, and/or the selected atomic structure. For example, the analysis unit 170 may analyze the structural features of the basic candidate structure, the modified atomic structure, and/or the selected atomic structure, by the principal component analysis. The output unit 150 may plot and output results of the principal component analysis, on a plane, as shown in Fig. 7 to Fig. 8. The output unit 150 may plot and output the results of the principal component analysis in a space having three or more dimensions. By executing S100 to S900 in this way, the single image analysis is executed.

The output unit 150 may output a result obtained by the evaluation unit 160 evaluating interactions between the basic candidate structure, the modified atomic structure, and/or the selected atomic structure. For example, the evaluation unit 160 may execute an electronic state calculation using a fragment molecular orbital (FMO) method on the basic candidate structure, the modified atomic structure, and/or the selected atomic structure obtained by the single image analysis.

In the FMO, the molecule is divided into subunits called fragments, and electronic energy obtained by the calculation for each fragment is used to calculate an electronic state of the molecule. For the FMO calculation, for example, it is possible to use known software such as ABINIT-MP [6-7] Open Ver. 1 Rev. 23Q2. The analysis of interactions between fragments may be performed by, for example, the pair-interaction energy decomposition analysis (PIEDA).

The evaluation unit 160 may calculate, as an interdomain IFIE map, interactions between fragments (residues) and/or between domains of the basic candidate structure, the modified atomic structure, and/or the selected atomic structure. For example, when the target biomolecule has six domains, the analysis unit 170 may generate a 6 × 6 tiled heatmap.

The evaluation unit 160 may analyze an interaction between a fragment included in a first domain and a fragment included in a second domain that is another domain, thereby calculating the interaction between the first domain and the second domain. For example, among interfragment interaction energies (for example, electrostatic interaction ES, exchange-repulsion interaction EX, charge-transfer interaction CT+mix, dispersion interaction DI, or the like) in the fragment molecular orbital method (FMO), the evaluation unit 160 may specify a fragment pair for which an absolute value of any of three components other than ES is greater than or equal to a predetermined value (for example, 3 kcal/mol). For example, the evaluation unit 160 may calculate the interaction between domains, by averaging the interactions of the specified fragment pair for each domain.

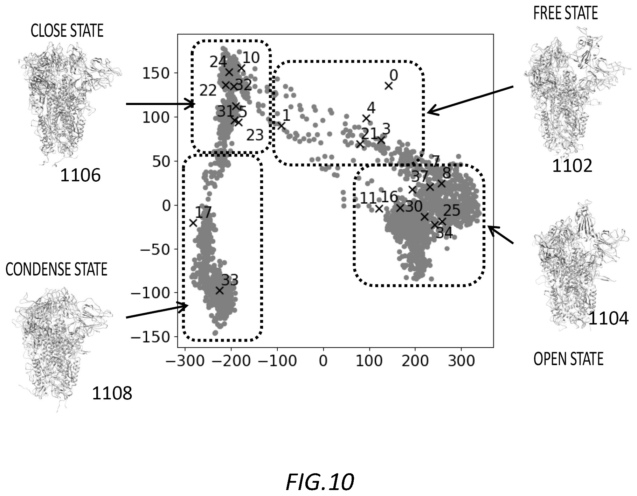

Fig. 10 shows an example of a state transition of the basic candidate structure, the modified atomic structure, and the selected atomic structure. The basic candidate structure, the modified atomic structure, and the selected atomic structure are mainly classified into four states which are a Free state 1102, an Open state 1104, a Close state 1106, and a Condense state 1108. Accordingly, it is considered that the target biomolecule transitions between these states. The evaluation unit 160 can evaluate, by performing the FMO calculation, degrees to which the interactions between the fragments (residues) and/or between the domains of the selected atomic structure contribute to stabilization of these states.

In S1000, the output unit 150 may perform the multi-image analysis. in S400 or S500, the global search unit 130 may attribute, based on the degree of similarity, each of the generation images to any of all or some of the representative basic candidate structures. For example, the global search unit 130 may attribute the generation image to one or more basic candidate structures having a high degree of similarity. In S700 or S800, the local structure search may attribute each of the generation images to all or some of the representative modified atomic structures, and/or any of the selected atomic structures. Here, the output unit 150 may display, on a plane or in a space, the number of attributions of the generation images for each of the basic candidate structure, the modified atomic structure, and/or the selected atomic structure.

For example, the output unit 150 may display the number of attributions as a numerical value on the plane or the space on which the results of the principal component analysis are plotted. For example, the output unit 150 may display the number of attributions, as a heatmap, on the plane or in the space on which the results of the principal component analysis are plotted. This makes it possible for the atomic structure estimation apparatus 10 to not only elucidate what kind of structures the target biomolecule can adopt, but also determine which states are stable (the number of attributions is greater), or the like.

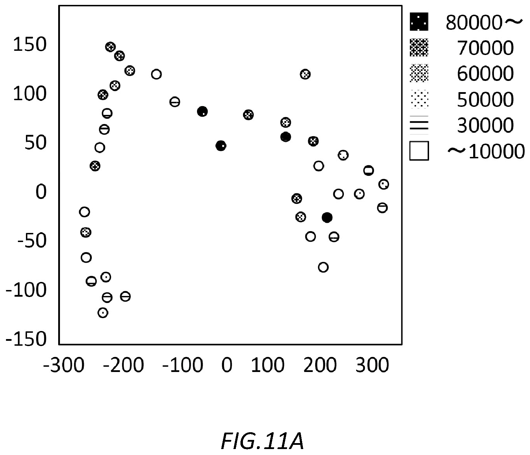

Fig. 11A shows an example in which the number of attributions of generation images is indicated as a heatmap. The output unit 150 may output the heatmap as shown in Fig. 11A. Each point in Fig. 11A corresponds to each of the basic candidate structures selected as being representative. Each point is displayed with different shading according to the degree of the number of attributions of generation images to the corresponding basic candidate structure. For example, the greater the number of attributions, the darker the point is displayed.

As shown in Fig. 11A, the generation images derived from one target biomolecule are attributed, based on the degree of similarity, to a plurality of representative structures different from each other. This shows that the basic candidate structure adopts the diverse states, and transitions between the states.

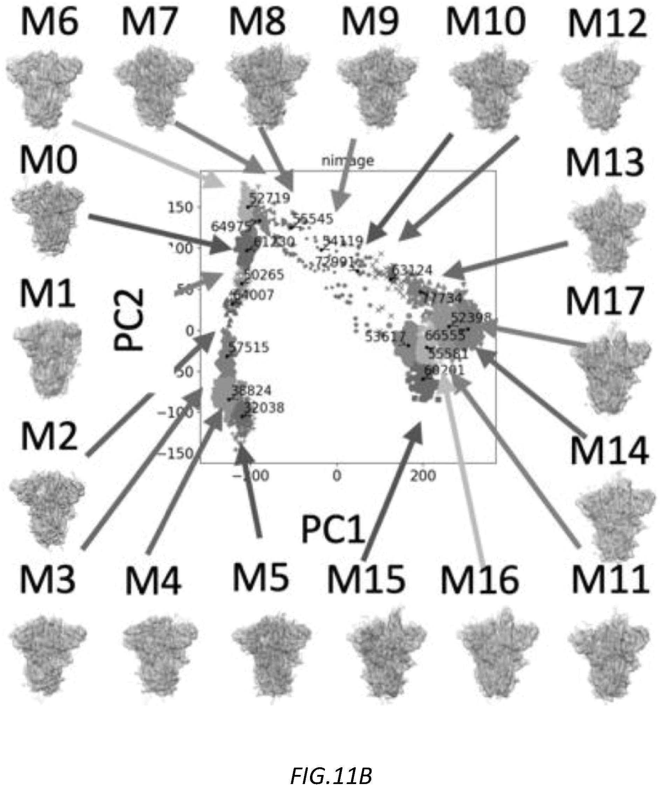

A three-dimensional density map may be constructed by performing three-dimensional reconstruction using a set of generation images attributed to the representative structure, or a set of the original particle images linked to the generation images. As shown in Fig. 11B, the three-dimensional reconstruction may be performed for each of the particle image sets attributed to the plurality of representative structures, thereby constructing the plurality of diverse three-dimensional density maps such as those shown by M0 to M17. Via the global search, it is possible to construct more diverse three-dimensional density maps in comparison with the cryoDRGN which is an existing method.

The output unit 150 may output the motion of the target biomolecule as the video. For example, the output unit 150 may express the motion of the target biomolecule as the video by continuously displaying three-dimensional structure models of a plurality of similar atomic structures or the generation images attributed thereto. As an example, the output unit 150 may display a structural transition of the target biomolecule, as the video, by continuously displaying adjacent basic candidate structures, modified atomic structures, and/or selected atomic structures (or the generation images attributed thereto), on the plane or in the space plotted by the principal component analysis or the like.

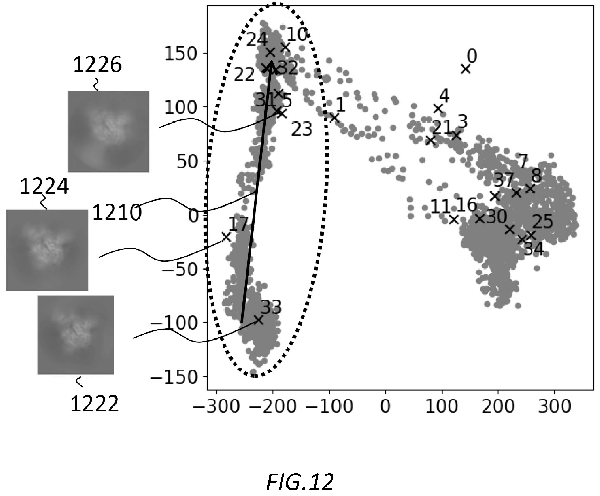

Fig. 12 shows an example of a video display by the output unit 150. For example, the output unit 150 acquires a generation image 1222 attributed to an adjacent atomic structure 33, a generation image 1224 attributed to an atomic structure 17, and a generation image 1226 attributed to an atomic structure 23, in the plane plotted by the principal component analysis.

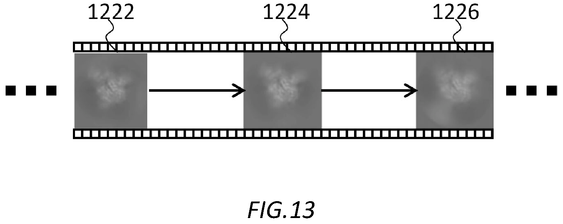

Next, for the output unit 150 as shown in Fig. 13, the output unit 150 displays the generation image 1222, the generation image 1224, and the generation image 1226 in order according to a positional relationship on a trajectory 1210. This makes it possible for the output unit 150 to pseudo-generate the video of the target biomolecule which gradually undergoes the state transition.

At least some of the flow from S100 to S1000 may be implemented by the parallel computation. For example, S200 and S400 to S700 may be executed in parallel by a supercomputer having a large number of computation nodes.

EXAMPLE

COMPLEX

The transmission electron microscope was used to perform an observation to capture an image by setting, as the target biomolecule, a Fab complex in which an infection-enhancing antibody (Fab) is bonded to a spike protein of SARS-CoV-2. The spike protein has three Fab bonding sites. Therefore, for the observed particle images, there may be a mix of cases where the number of bonded Fabs is 0, 1, 2, and 3. Based on the captured image data, the CryoEM particle image set (approximately 110,000 images, J326) of the Fab complex of the SARS-CoV-2 spike protein was acquired by using dedicated software RELION.

The generation image with improved quality was obtained by applying the VAE (latent space dimension 12, Dropout = 0.3, noise = 0.3) to the CryoEM particle image set. Further, clustering analysis by an X-means method was performed on each improved image, in a VAE latent space, and a representative image of each cluster was obtained. 15 clusters were obtained; however, two clusters of which the representative images were not clear and which were widely distributed in the latent space were excluded, and 13 representative images of the clusters were selected as the target images to undergo subsequent processing.

In parallel, the virtual molecule was prepared by the MD. The CryoEM images of the Fab complex can be a mixture of the Fab complex to which no Fab is bonded (set as "Fab 0"), the Fab complex to which one Fab is bonded (set as "Fab 1"), the Fab complex to which two Fabs are bonded (set as "Fab 2"), and the Fab complex to which three Fabs are bonded (referred to as "Fab 3"). Therefore, four patterns of the structural space were prepared in advance by an all-atom version OFLOOD (AA-OFLOOD). For each bonding state, the AA-OFLOOD was performed with 250 replicas for 20 cycles, and for each Fab state, structure sampling was performed with an upper limit of 500,000 structures that are stored being set. The principal component analysis was performed on 500,000 structures, and approximately 5,000 representative structures were selected on the PC1-PC2 plane.

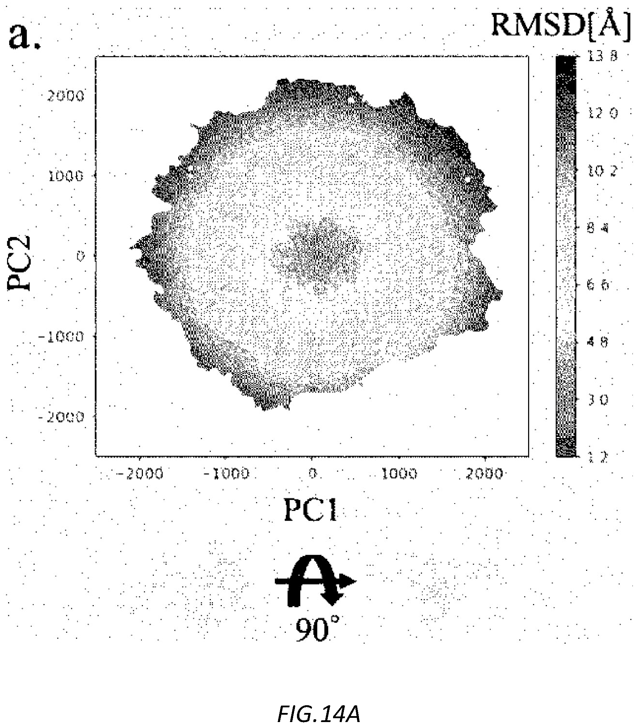

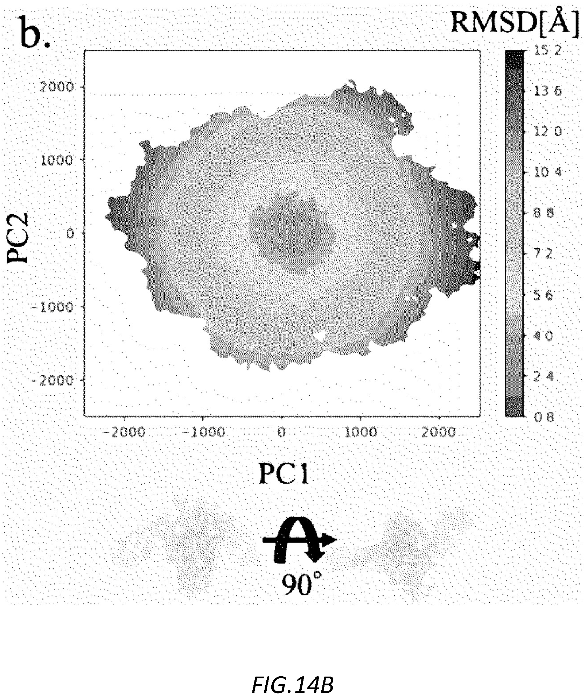

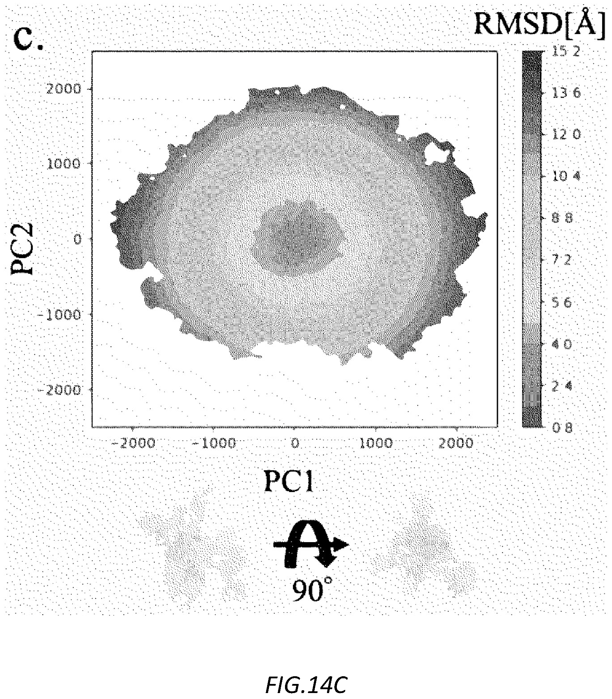

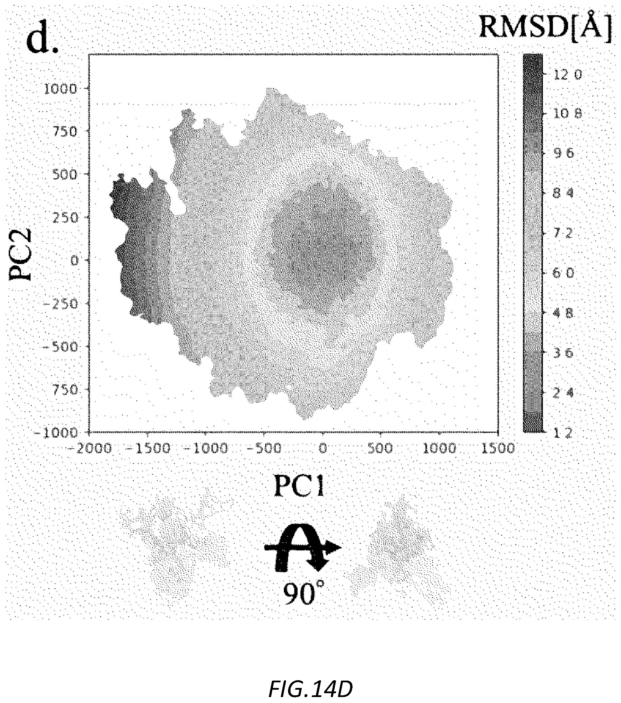

As shown in Fig. 14A to Fig. 14B (hereinafter collectively referred to as Fig. 14), a diverse structural space was constructed with a maximum RMSD of 15 angstroms based on the initial structure. In Fig. 14, the structural distributions of the structural spaces and structure examples of Fab 0 to Fab 3 were projected onto the principal component axes (PC1-PC2), and the RMSD values from the initial structure were represented by shading.

The degree of similarity was calculated by the method described in S400 to S500 and the like, and the basic candidate structure was selected. Specifically, for each 3D atomic structure generated by the MD, the similarity scores were comprehensively calculated between a set of 2D projection images from all orientations (which is obtained by dividing 180 degrees into 10 to 240 segments in consideration of symmetry in the Fourier space) and experimental 2D images with improved quality, thereby selecting a 3D atomic model showing the highest similarity score as a Promising structure (that is, the basic candidate structure).

The local structure search was executed by the method described in S600 to S800, or the like. Specifically, by setting the Promising structure obtained by the global search as the initial structure, and performing the local search on a structure that matches the target image more closely in its vicinity with 100 cycles of the PaCS (Parallel Cascade Selection)-MD, a final model structure was obtained. In this local search, a Modified PaCS-MD algorithm in which the similarity score in the FT image is set as a constraint condition, was applied, thereby efficiently searching for the three-dimensional structure showing a higher similarity score.

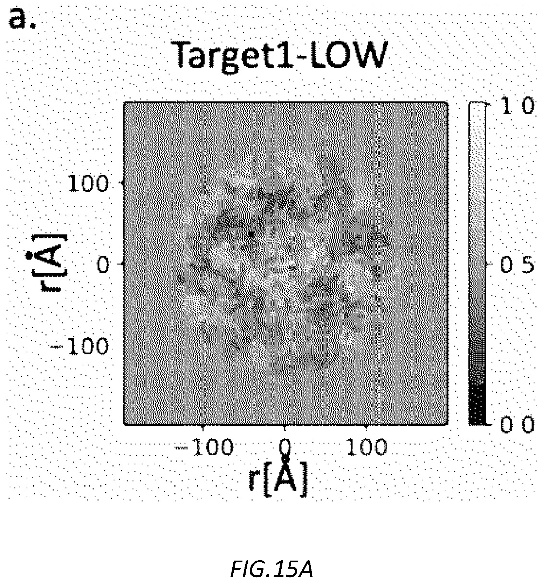

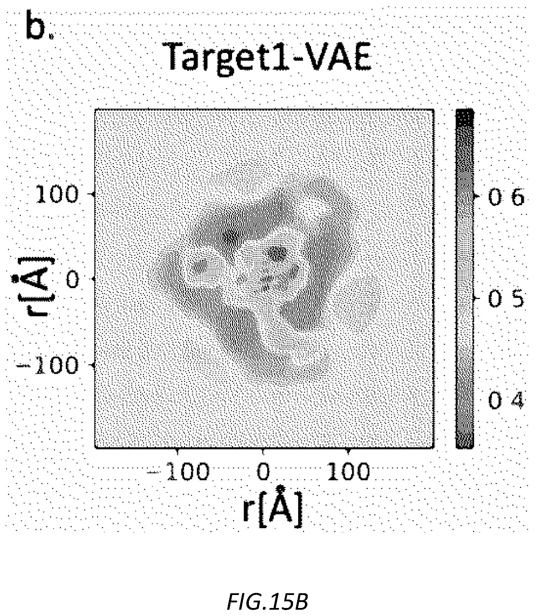

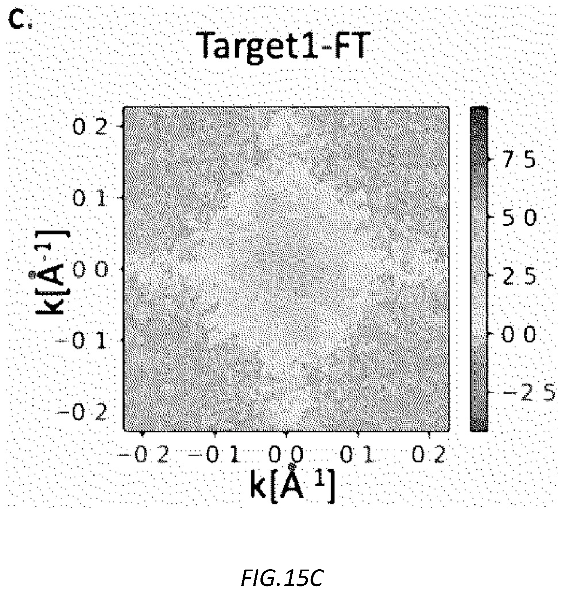

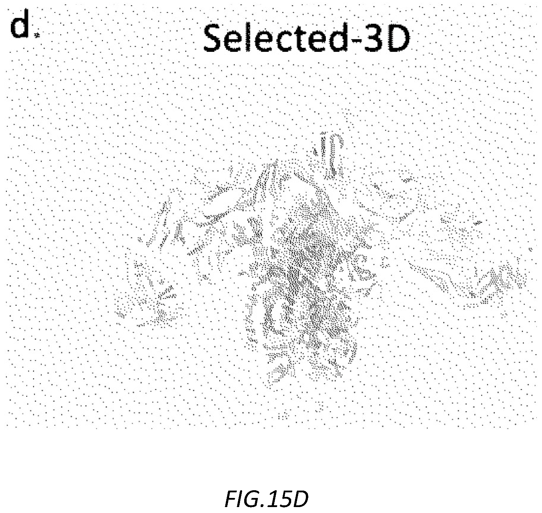

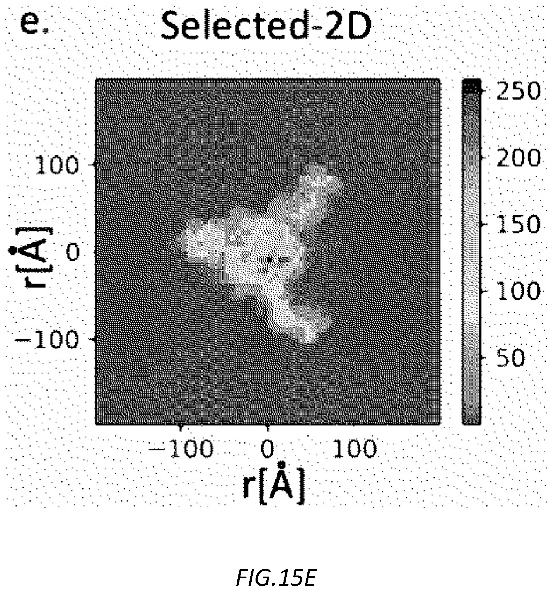

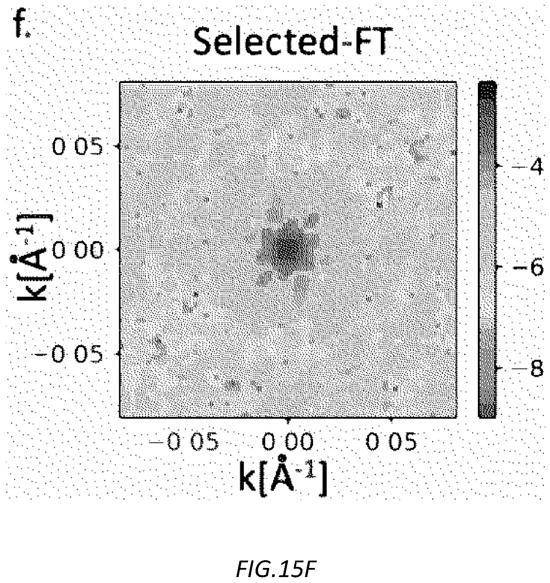

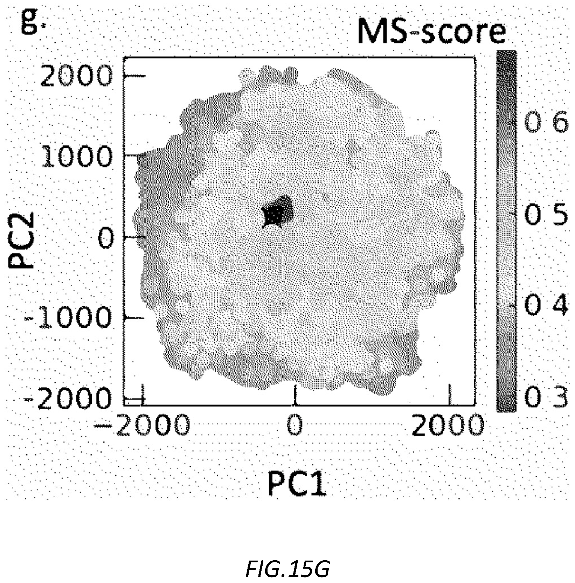

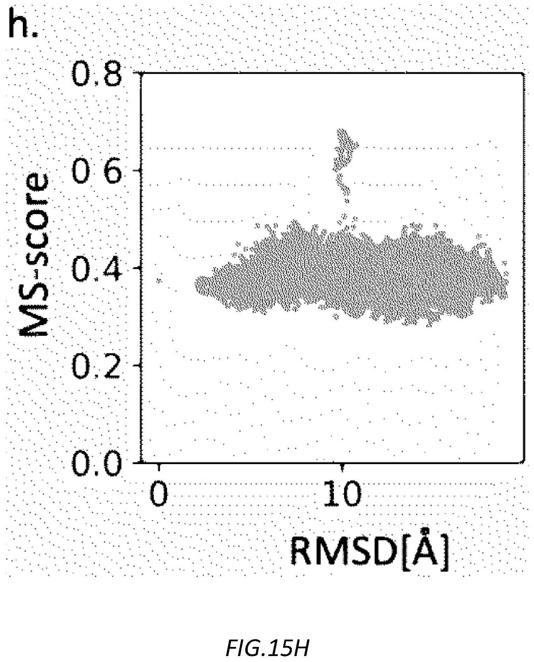

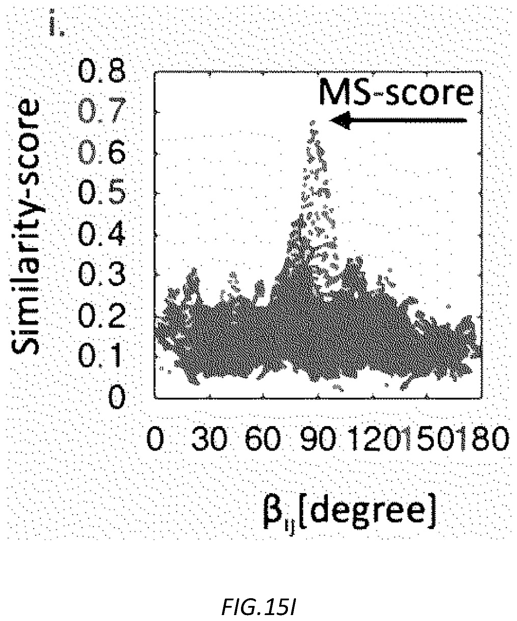

Fig. 15A to Fig. 15I (hereinafter collectively referred to as Fig. 15) show the search results obtained for Fab 3 in which three antibodies are bonded to the spike protein. As shown in Fig. 15G to Fig. 15H, it was possible to specify a plausible three-dimensional structure showing the degree of similarity of approximately 0.7. Target 1 in Fig. 15 corresponds to one cluster of 15 clusters (Target 0 to Target 14).

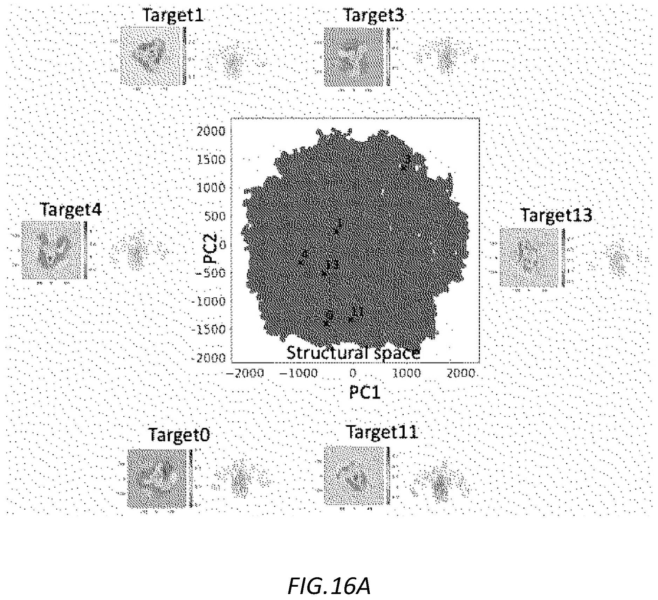

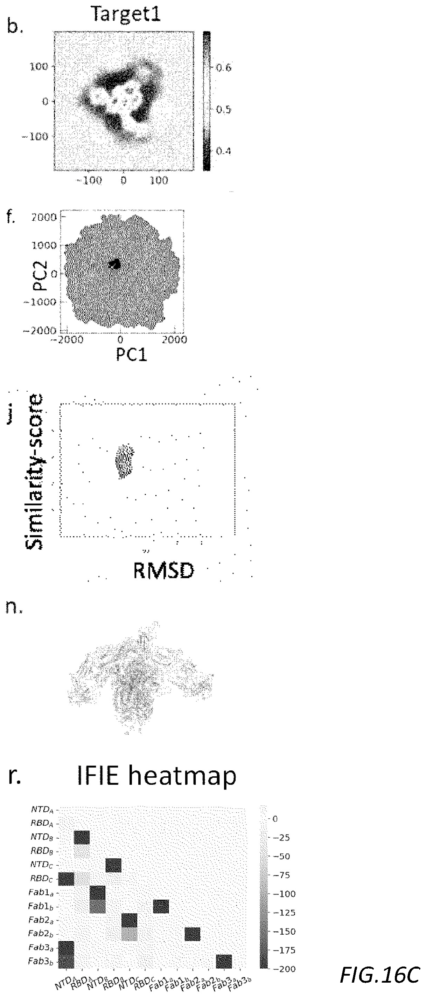

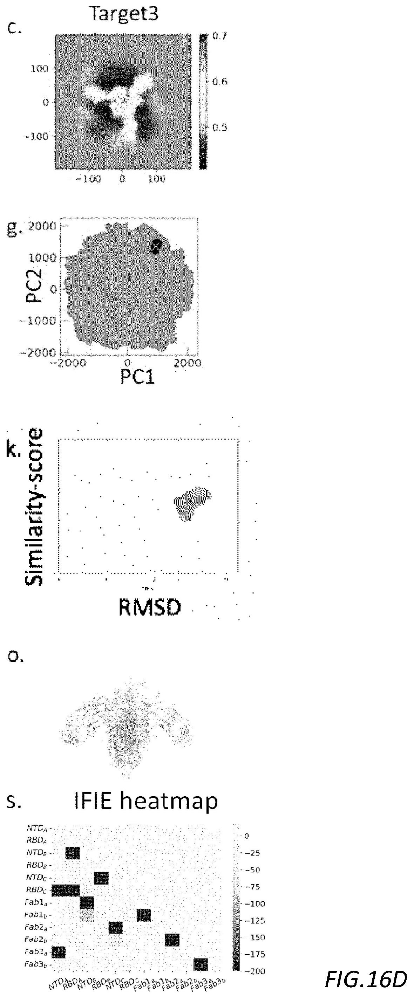

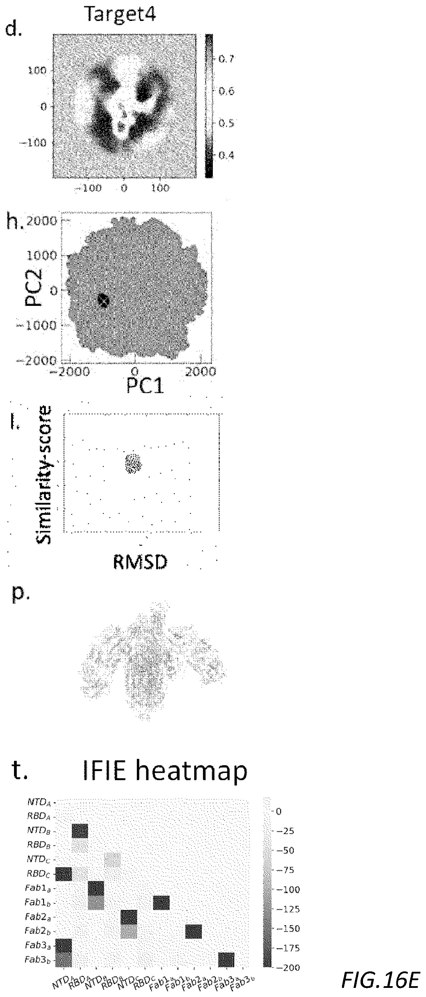

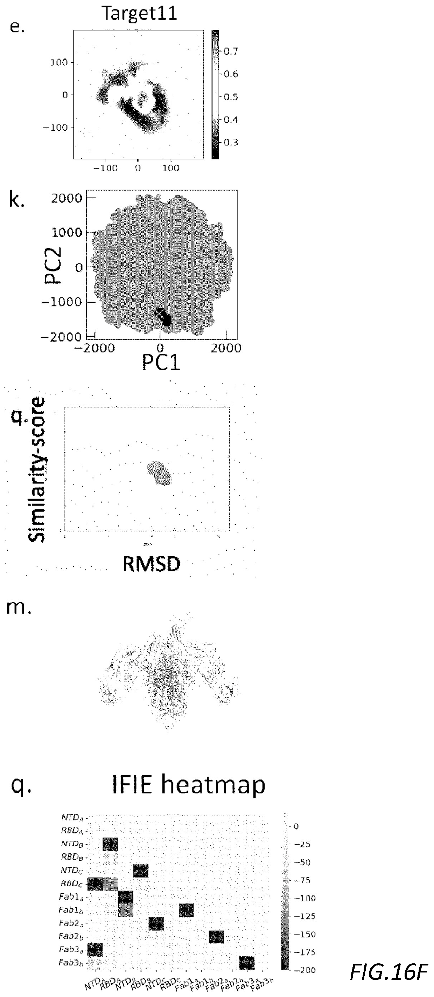

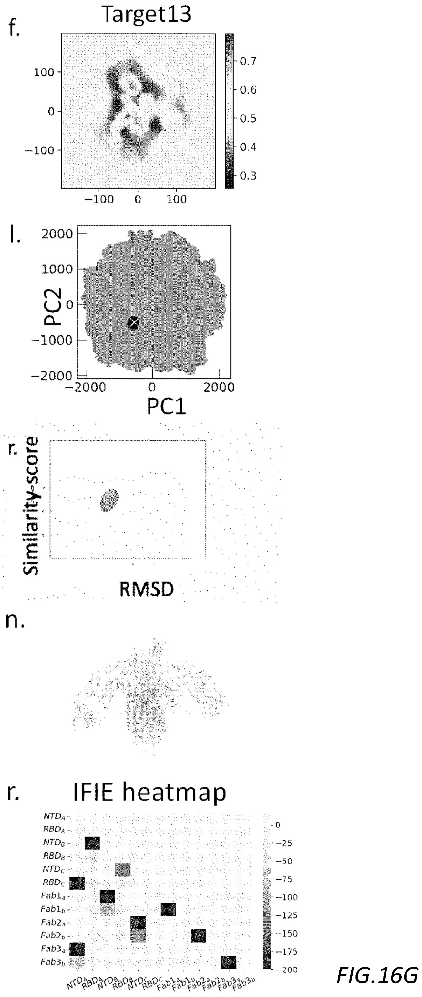

As a result of performing the global structure search on the 13 target images in which noise was reduced, six clusters (Targets 0, 1, 3, 4, 11, 13) were found to have high likelihood of corresponding to Fab 3, three clusters (Targets 2, 6, 9) were found to have high likelihood of corresponding to Fab 2, and four clusters (Targets 5, 7, 10) were found to have high likelihood of corresponding to Fab 1, and no cluster was found to have high likelihood of corresponding to Fab 0. Fig. 16A shows estimated structural positions of each cluster corresponding to Fab 3, in the structural space (PC1-PC2 plane), and the estimated three-dimensional structures.

The electronic state was evaluated by the method described in the S1000 or the like. The electronic state calculation using the fragment molecular orbital (FMO) method was performed on the structure having the top-ranked degree of similarity.

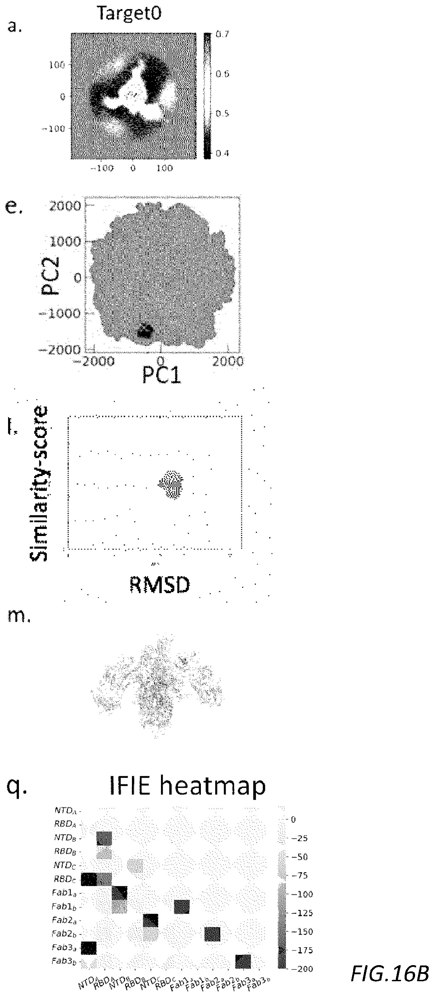

Fig. 16B to Fig. 16G show the results of the local structure search performed on the representative clusters (Targets 0, 1, 3, 4) together with domain IFIE map diagrams obtained by the FMO calculation. As illustrated, the Close state (Target 1 shown as r) and the Condense state (Target 3 shown as s) were observed. The complex-specific patterns that were not observed in the spike protein alone which will be described below, were also observed (Targets 0, 4). In the interaction with a Fab region, a case where not only a region of $Fab_a$ but also a region of $Fab_b$ simultaneously interacts with an NTD, was observed (Targets 1, 4).

SPIKE PROTEIN ALONE

The transmission electron microscope was used to perform an observation to capture an image by setting, as the target biomolecule, the wild type and the mutant type (D614G) of the SARS-CoV-2 spike protein. Based on the captured image data, the CryoEM particle image set (approximately 190,000 images) of the wild type and the CryoEM particle image set (approximately 120,000 images) of the mutant type were acquired by using the dedicated software RELION.

The generation image with improved quality was obtained by applying the VAE (latent space dimension 12, Dropout = 0.3, noise = 0) to the CryoEM particle image set. Further, clustering analysis by a k-means method was performed on each improved image, and 38 clusters of the wild type and 36 clusters of the mutant type were obtained. Based on the representative images of the clusters and the properties (widely distributed or narrowly distributed) of the distribution of each cluster in the latent space, 26 target images of the wild type and 26 target images of the mutant type were selected.

The single image analysis was applied to 26 target image sets of the wild type; and the FMO calculation, and analyses by the IFIE and the PIEDA were performed on Top 10 structure sets (a sum of 260 structures) estimated from each of the target images. It was found that four characteristic interaction states existed. Each state was set as the Open state, the Close state, the Ccondense state, or the free state.

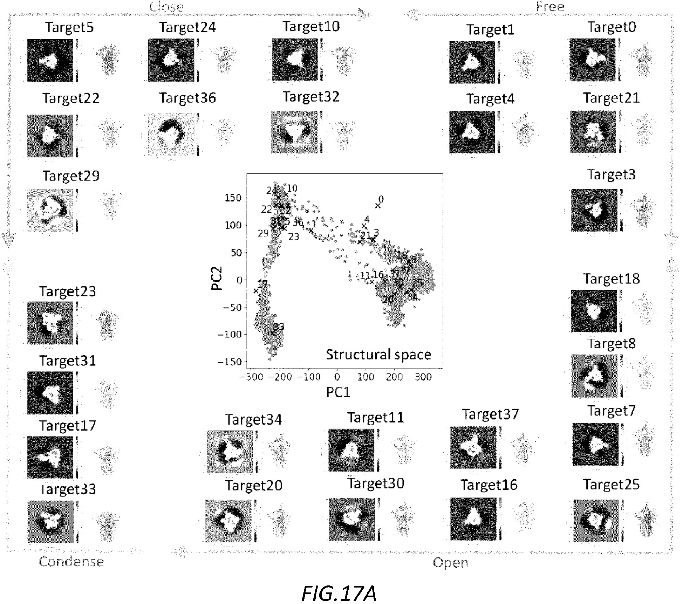

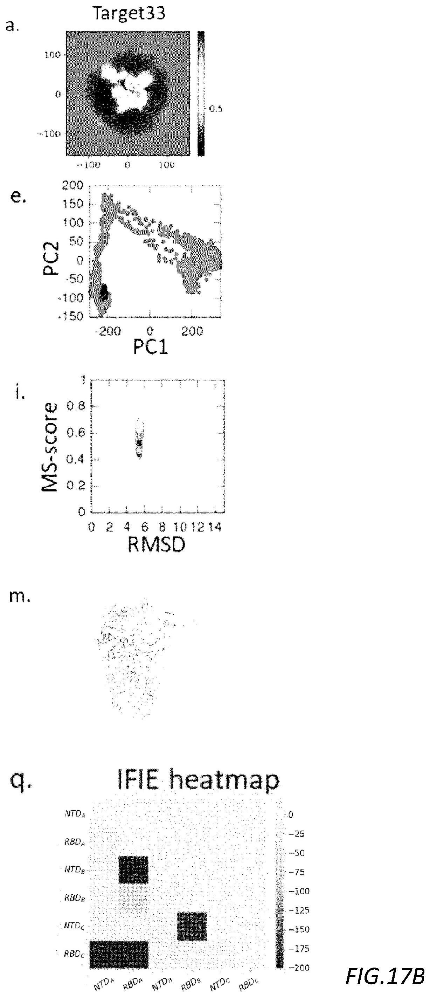

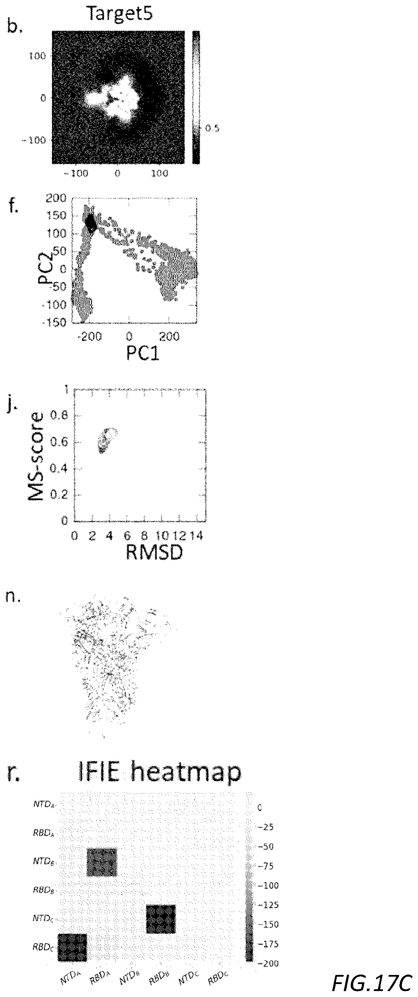

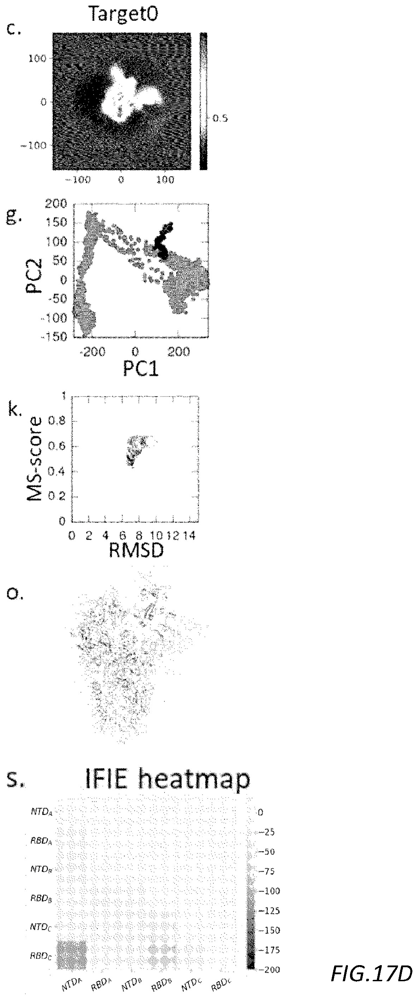

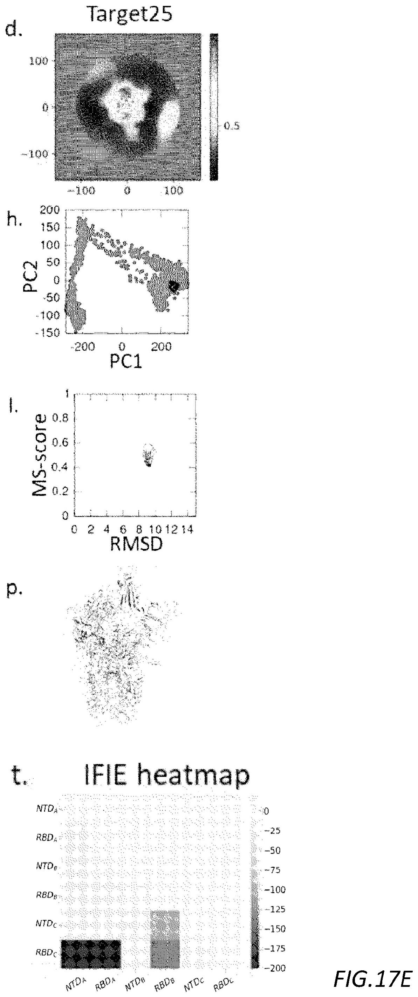

Fig. 17A shows the estimated structural position in the structural space (PC1-PC2 plane) for the wild type, and the target image and the estimated three-dimensional structure. Fig. 17B to Fig. 17E show the results of the local search for representative four cases (Targets 33, 5, 0, 25) in the wild type, and the domain IFIE map diagrams or the like obtained by the FMO calculation. The interdomain IFIE map is constitutes by $6 \times 6$ tiles, and the darker the color of each tile, the more stably the interaction occurs between the domains.

The single image analysis was applied to 26 target image sets of the mutant type, as well; and the FMO calculation, and analyses by the IFIE and the PIEDA were performed on Top 10 structure sets (a sum of 260 structures) estimated from each of the target images. The success rate of the FMO calculation was 78.8% (205 successful cases out of 260 cases). The Free state had a high ratio of 46%, suggesting that a shift in structural state occurred due to the mutation. It was found that the D614G mutation stabilizes the structural state with an RBD up (Open), and tends to enhance viral infection.

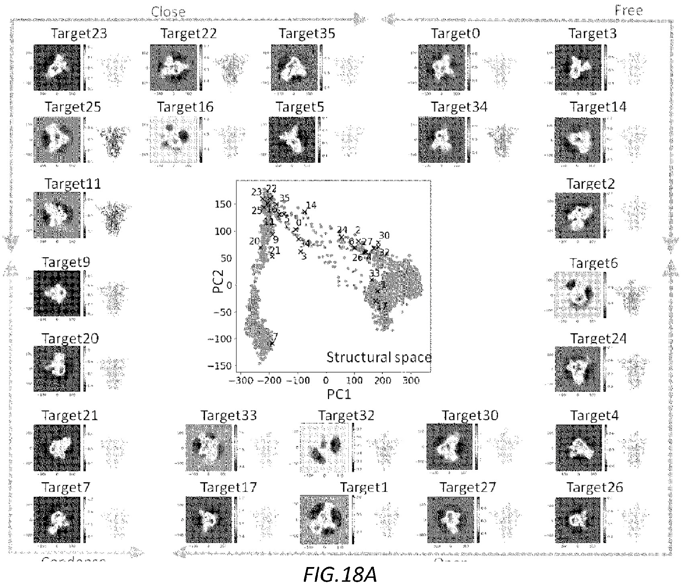

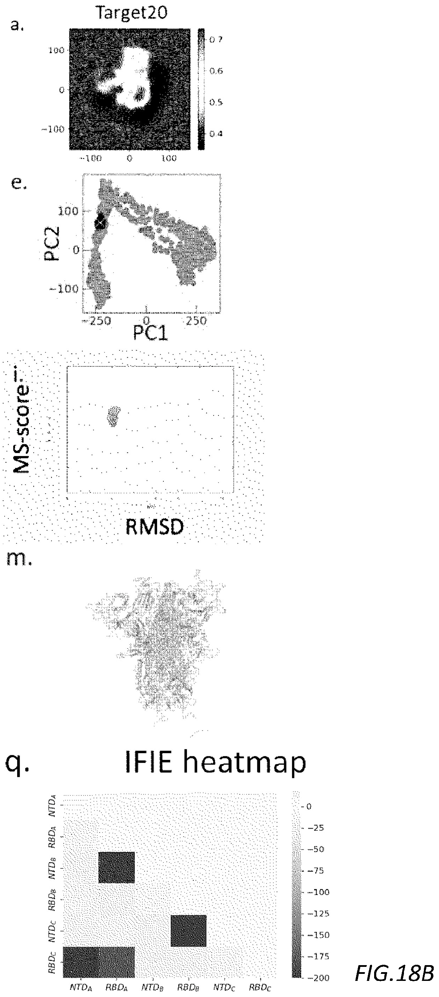

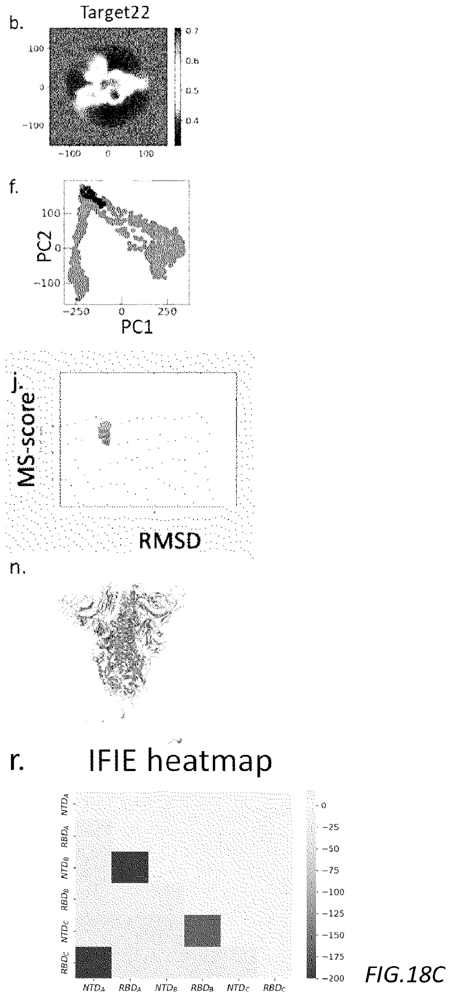

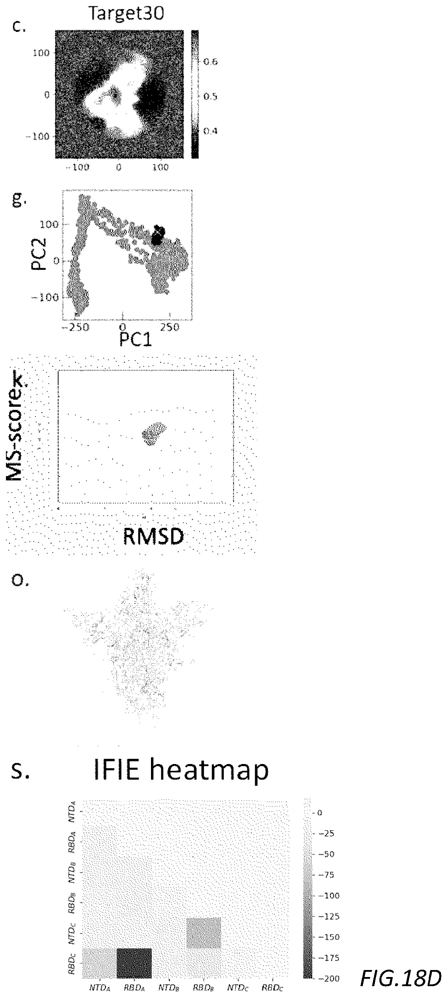

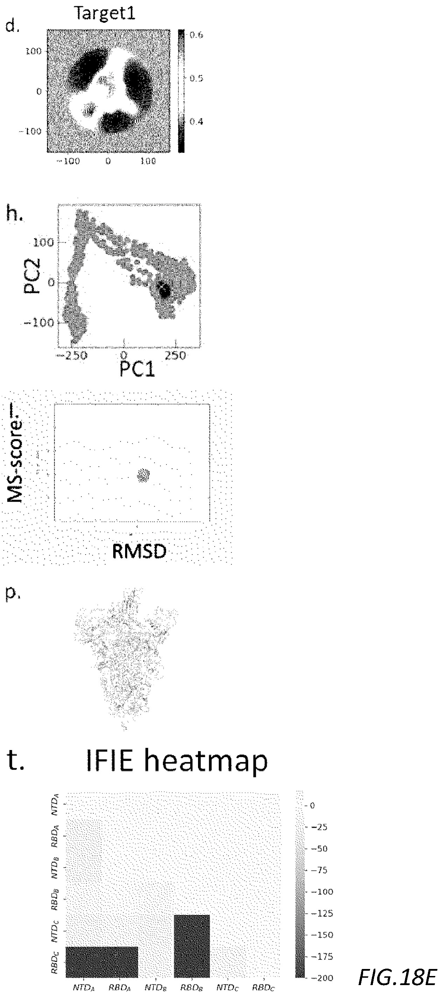

Fig. 18A shows the estimated structural position in the structural space (PC1-PC2 plane) for the mutant type, and the target image and the estimated three-dimensional structure. Fig. 18B to Fig. 18E show the results of the local search for the representative four cases (Targets 20, 22, 30, 1) in the mutant type, and the domain IFIE map diagrams or the like obtained by the FMO calculation.

In this way, with the present embodiment, by implementing the single image analysis, it is possible to: specify, with fewer computational resources and with high precision, the atomic structures of a plurality of structural states of the target biomolecule, based on experiment data, through the analyses in the image space and the structural space; and output the specified atomic structures as the three-dimensional structures. With the present embodiment, it is possible to elucidate the diverse structures of the target biomolecule. With the present embodiment, it is possible to elucidate and output the state transitions between the structures and the interactions between the fragments, and the like. With the present embodiment, it was possible to elucidate diverse structural states of the biomolecule with a high resolution based on the experimental data. In addition to or instead of this, it was also possible to evaluate and/or output the diverse elucidated structural states by using a unique method.

Various embodiments of the present invention may be described with reference to flowcharts and block diagrams, where blocks may represent (1) stages of processes in which operations are executed or (2) sections of apparatuses responsible for executing operations. Certain stages and sections may be implemented by a dedicated circuit, a programmable circuit supplied together with computer-readable instructions stored on computer-readable media, and/or processors supplied together with computer-readable instructions stored on computer-readable media. The dedicated circuit may include digital and/or analog hardware circuits, and may include integrated circuits (IC) and/or discrete circuits. The programmable circuit may include a reconfigurable hardware circuit including logical AND, logical OR, logical XOR, logical NAND, logical NOR, and other logical operations, a memory element or the like such as a flip-flop, a register, a field programmable gate array (FPGA) and a programmable logic array (PLA), or the like.

A computer-readable medium may include any tangible device that can store instructions to be executed by a suitable device, and as a result, the computer-readable medium having instructions stored thereon includes a product including instructions that can be executed in order to create means for executing operations specified in the flowcharts or block diagrams. Examples of the computer-readable medium may include an electronic storage medium, a magnetic storage medium, an optical storage medium, an electromagnetic storage medium, a semiconductor storage medium, or the like. More specific examples of the computer-readable medium may include a floppy (registered trademark) disk, a diskette, a hard disk, a random access memory (RAM), a read-only memory (ROM), an erasable programmable read-only memory (EPROM or flash memory), an electrically erasable programmable read-only memory (EEPROM), a static random access memory (SRAM), a compact disc read-only memory (CD-ROM), a digital versatile disc (DVD), a Blu-ray (registered trademark) disc, a memory stick, an integrated circuit card, and the like.

The computer-readable instruction may include: an assembler instruction, an instruction-set-architecture (ISA) instruction; a machine instruction; a machine dependent instruction; a microcode; a firmware instruction; state-setting data; or either a source code or an object code written in any combination of one or more programming languages, including an object oriented programming language such as Smalltalk (registered trademark), JAVA (registered trademark), C++, or the like, and a conventional procedural programming language such as a "C" programming language or a similar programming language.

The computer-readable instruction may be provided to a processor of a programmable data processing apparatus such as a computer, or to a programmable circuit, locally or via a local area network (LAN), wide area network (WAN) such as the Internet, or the like, to execute the computer-readable instructions in order to create means for performing operations specified in the flowcharts or block diagrams.

Here, the computer may be a computer such as a personal computer (PC), a tablet type computer, a smartphone, a workstation, a server computer, cloud server, or a general purpose computer, or may be a computer system in which a plurality of computers are connected to each other. Such a computer system in which the plurality of computers are connected to each other is also called a distributed computing system, and is a computer in a broad sense. In the distributed computing system, the plurality of computers collectively execute the program by each of the plurality of computers executing a part of the program, and passing data during the execution of the program among the computers as needed.

Examples of the processor include a computer processor, a central processing unit (CPU), a processing unit, a microprocessor, a digital signal processor, a controller, a microcontroller, or the like. The computer may include one processor or a plurality of processors. In a multiprocessor system including a plurality of processors, the plurality of processors collectively execute a program by each of the processors executing a part of the program, and passing data during the execution of the program among the processors as needed. For example, in execution of multiple tasks, each of the plurality of processors may execute a portion of each task piece by piece by performing task-switching for each time slice. In this case, which portion of one program each processor is responsible for executing dynamically changes. In addition, which portion of the program each of the plurality of processors is to execute may be statically determined by multi-processor aware programming.

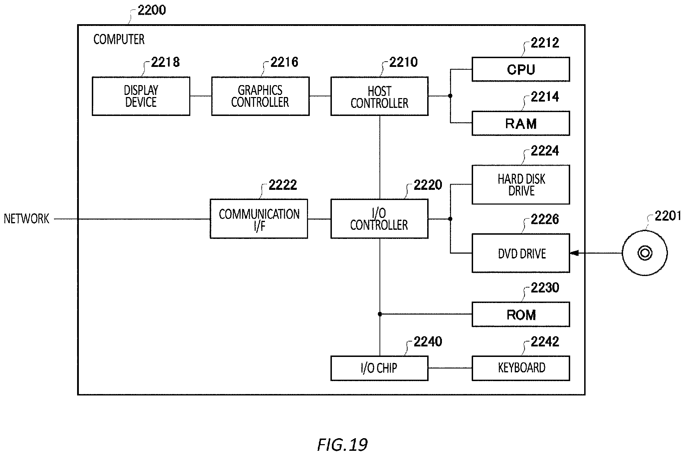

Fig. 19 shows an example of a computer 2200 in which a plurality of aspects of the present invention may be embodied entirely or partially. A program installed in the computer 2200 can cause the computer 2200 to function as an operation associated with the apparatuses according to the embodiments of the present invention or as one or more sections of the apparatuses, or can cause the operation or the one or more sections to be executed, and/or can cause the computer 2200 to execute a process according to the embodiments of the present invention or a step of the process. Such programs may be executed by a CPU 2212 to cause the computer 2200 to perform specific operations associated with some or all of the blocks in the flowcharts and block diagrams described in the present specification.

The computer 2200 according to the present embodiment includes the CPU 2212, a RAM 2214, a graphics controller 2216, and a display device 2218, which are interconnected by a host controller 2210. The computer 2200 also includes input/output units such as a communication interface 2222, a hard disk drive 2224, a DVD-ROM drive 2226, and an IC card drive, which are connected to the host controller 2210 via an input/output controller 2220. The computer also includes legacy input/output units such as a ROM 2230 and a keyboard 2242, which are connected to the input/output controller 2220 via an input/output chip 2240.

The CPU 2212 operates according to programs stored in the ROM 2230 and the RAM 2214, thereby controlling each unit. The graphics controller 2216 acquires image data generated by the CPU 2212 in a frame buffer or the like provided in the RAM 2214 or in itself such that the image data is displayed on the display device 2218.

The communication interface 2222 communicates with other electronic devices via a network. The hard disk drive 2224 stores programs and data used by the CPU 2212 in the computer 2200. The DVD-ROM drive 2226 reads the programs or the data from a DVD-ROM 2201, and provides the programs or the data to the hard disk drive 2224 via the RAM 2214. The IC card drive reads the programs and the data from the IC card, and/or writes the programs and the data to the IC card.

The ROM 2230 stores therein boot programs and the like executed by the computer 2200 at the time of activation, and/or programs that depend on the hardware of the computer 2200. The input/output chip 2240 may also connect various input/output units to the input/output controller 2220 via a parallel port, a serial port, a keyboard port, a mouse port, or the like.

Programs are provided by a computer-readable medium such as the DVD-ROM 2201 or the IC card. The programs are read from the computer-readable medium, are installed in the hard disk drive 2224, the RAM 2214, or the ROM 2230 which is also an example of the computer-readable medium, and are executed by the CPU 2212. The information processing written in these programs is read by the computer 2200, and provides cooperation between the programs and the various types of hardware resources. The apparatus or method may be constituted by implementing operations or processing of information according to use of the computer 2200.

For example, in a case where communication is performed between the computer 2200 and an external device, the CPU 2212 may execute a communication program loaded in the RAM 2214 and instruct the communication interface 2222 to perform communication processing based on processing written in the communication program. Under the control of the CPU 2212, the communication interface 2222 reads transmission data stored in a transmission buffer processing region provided in a recording medium such as the RAM 2214, the hard disk drive 2224, the DVD-ROM 2201, or the IC card, transmits the read transmission data to the network, or writes reception data received from the network in a reception buffer processing region or the like provided on the recording medium.

In addition, the CPU 2212 may cause the RAM 2214 to read all or a necessary part of a file or database stored in an external recording medium such as the hard disk drive 2224, the DVD-ROM drive 2226 (DVD-ROM 2201), the IC card, or the like, and may execute various types of processing on data on the RAM 2214. Then, the CPU 2212 writes the processed data back in the external recording medium.

Various types of information such as various types of programs, data, tables, and databases may be stored in a recording medium and subjected to information processing. The CPU 2212 may execute, on the data read from the RAM 2214, various types of processing including various types of operations, information processing, conditional judgement, conditional branching, unconditional branching, information retrieval/replacement, or the like described throughout the present disclosure and specified by instruction sequences of the programs, to write the results back to the RAM 2214. In addition, the CPU 2212 may retrieve information in a file, a database, or the like in the recording medium. For example, when a plurality of entries, each having an attribute value of a first attribute associated with an attribute value of a second attribute, is stored in the recording medium, the CPU 2212 may retrieve, out of the plurality of entries, an entry with the attribute value of the first attribute specified that meets a condition, read the attribute value of the second attribute stored in the entry, and thereby acquiring the attribute value of the second attribute associated with the first attribute meeting a predetermined condition.

The programs or software modules described above may be stored in a computer-readable medium on the computer 2200 or near the computer 2200. In addition, a recording medium such as a hard disk or a RAM provided in a server system connected to a dedicated communication network or the Internet can be used as a computer-readable medium, thereby providing a program to the computer 2200 via the network.

While the embodiments of the present invention have been described, the technical scope of the present invention is not limited to the above-described embodiments. It is apparent to persons skilled in the art that various alterations or improvements can be added to the above-described embodiments. It is also apparent from the described scope of the claims that the embodiments to which such alterations or improvements are added can be included in the technical scope of the present invention.

Note that the operations, procedures, steps, stages, or the like of each process performed by a device, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order. The notation "A and/or B" may indicate A, B, or A and B. The expression "A, B, and/or C" may indicate "any one of A, B, and C, or any combination of two or more thereof."

EXPLANATION OF REFERENCES

10 atomic structure estimation apparatus
110 generation unit
120 acquisition unit
130 global search unit
140 local search unit
150 output unit
160 evaluation unit
170 analysis unit
402 virtual molecule

602 first candidate structure image
604 first candidate structure image
606 first candidate structure image
702 basic candidate structure
802 selected atomic structure
902 three-dimensional structure model
904 three-dimensional structure model
1102 Free state
1104 Open state
1106 Close state
1108 Condense state
1210 trajectory
1222 generation image
1224 generation image
1226 generation image.

**Claims**

1. An atomic structure estimation apparatus for a biomolecule, comprising:

   a generation unit which generates a generation image based on a target biomolecule;
   an acquisition unit which acquires, from a plurality of prepared atomic structures, a plurality of first candidate structure images based on two-dimensional projection images;
   a global search unit which selects, as a basic candidate structure, an atomic structure that has a high degree of similarity to the target biomolecule in structure and/or molecular orientation, from among a plurality of atomic structures, based on a degree of similarity between the generation image and a first candidate structure image which is included in the plurality of first candidate structure images; and
   a local search unit which acquires a plurality of second candidate structure images based on two-dimensional projection images of a plurality of modified atomic structures obtained by modifying the basic candidate structure, and selects an atomic structure that has a high degree of similarity to the target biomolecule in structure and/or molecular orientation, from among the modified atomic structures, based on a degree of similarity between a second candidate structure image which is included in the plurality of second candidate structure images and the generation image.

2. The atomic structure estimation apparatus according to claim 1, wherein
   the acquisition unit acquires the plurality of first candidate structure images, based on two-dimensional projection images projected from a plurality of orientations surrounding an atomic structure which is included in the plurality of prepared atomic structures.

3. The atomic structure estimation apparatus according to claim 1, wherein
   the acquisition unit acquires the first candidate structure image, based on a two-dimensional projection image of an atomic structure which is included in the plurality of prepared atomic structures and which is reproduced by a molecular dynamics simulation.

4. The atomic structure estimation apparatus according to claim 1, wherein
   the local search unit increases a degree of similarity to the target biomolecule, by repeatedly modifying the basic candidate structure a plurality of times while maintaining a biomolecular structure.

5. The atomic structure estimation apparatus according to claim 1, wherein
   the local search unit simulates a structural transition of a modified atomic structure which is included in the plurality of modified atomic structures, by a cascade selection-based molecular dynamics simulation (PaCS-MD).

6. The atomic structure estimation apparatus according to claim 2, wherein
   the global search unit matches the plurality of first candidate structure images with each of a plurality of generation images which is the generation image, and selects the basic candidate structure based on a degree of similarity that is a matching result.

7. The atomic structure estimation apparatus according to claim 6, wherein
the global search unit selects the basic candidate structure based on a highest value, a lowest value, an average value, and/or a median value of degrees of similarity of the plurality of first candidate structure images matched with each of the plurality of generation images.

8. The atomic structure estimation apparatus according to claim 1, wherein
the global search unit computes the degree of similarity between the generation image and the first candidate structure image by a parallel computation.

9. The atomic structure estimation apparatus according to claim 1, wherein

the generation unit generates the generation image based on a particle image of the target biomolecule,
the generation unit reduces noise in the particle image, and
the generation image is a particle image in which the noise is reduced.

10. The atomic structure estimation apparatus according to claim 9, wherein
the generation unit reduces the noise in the particle image by an image generation by machine learning.

11. The atomic structure estimation apparatus according to claim 10, wherein

the generation unit applies a Fourier transform to the particle image in which the noise is reduced, and generates, as the generation image, a target wavenumber-space image that is an image after the Fourier transform, and
the acquisition unit applies the Fourier transform to a two-dimensional projection image of an atomic structure, and acquires, as the first candidate structure image, a wavenumber-space image that is the image after the Fourier transform.

12. The atomic estimation apparatus according to claim 11, wherein
the generation unit excludes a set of irrelevant images in a latent space.

13. The atomic structure estimation apparatus according to claim 1, further comprising:
an output unit which outputs a three-dimensional structure model of a modified atomic structure which is included in the plurality of modified atomic structures and which is selected by the local search unit.

14. The atomic structure estimation apparatus according to claim 1, further comprising:
an evaluation unit which evaluates and outputs interactions between fragments (residues) and/or between domains of a modified atomic structure which is included in the plurality of modified atomic structures and which is selected by the local search unit.

15. The atomic structure estimation apparatus according to claim 13, further comprising:

an analysis unit which analyzes structural features of the basic candidate structure and/or the modified atomic structure selected by the local search unit, by a principal component analysis, wherein
the output unit plots and outputs results of the principal component analysis, on a plane or in a space.

16. The atomic structure estimation apparatus according to claim 15, wherein

the global search unit attributes, based on the degree of similarity, the generation image to any of basic candidate structures, each of which is the basic candidate structure, and
the output unit further displays, on the plane or in the space, a number of at least one attribution of the generation image for each of the basic candidate structures.

17. The atomic structure estimation apparatus according to claim 16, wherein
the output unit displays the number of attributions, as a heatmap, on the plane or in the space.

18. The atomic structure estimation apparatus according to claim 16, wherein
the output unit displays a structural transition of the target biomolecule, as a video, by continuously displaying the generation image attributed to the basic candidate structure which is adjacent on the plane or in the space.

**19.** An atomic structure estimation method for a biomolecule, comprising:

generating a generation image based on a target biomolecule;
acquiring, from a plurality of prepared atomic structures, a plurality of first candidate structure images based on two-dimensional projection images;
performing a global search by selecting, as a basic candidate structure, an atomic structure that has a high degree of similarity to the target biomolecule in structure and/or molecular orientation, from among a plurality of atomic structures, based on a degree of similarity between the generation image and a candidate structure image which is included in the plurality of first candidate structure images; and
performing a local search by acquiring a plurality of second candidate structure images based on two-dimensional projection images of a plurality of modified atomic structures obtained by modifying the basic candidate structure, and selecting an atomic structure that has a high degree of similarity to the target biomolecule in structure and/or molecular orientation, from among the modified atomic structures, based on a degree of similarity between a second candidate structure image which is included in the plurality of second candidate structure images and the generation image.

**20.** A program which is executed by a computer, and causes the computer to function as:

a generation unit which generates a generation image based on a target biomolecule;
an acquisition unit which acquires, from a plurality of prepared atomic structures, a plurality of first candidate structure images based on two-dimensional projection images;
a global search unit which selects, as a basic candidate structure, an atomic structure that has a high degree of similarity to the target biomolecule in structure and/or molecular orientation, from among a plurality of atomic structures, based on a degree of similarity between the generation image and a candidate structure image which is included in the plurality of first candidate structure images; and
a local search unit which acquires a plurality of second candidate structure images based on two-dimensional projection images of a plurality of modified atomic structures obtained by modifying the basic candidate structure, and selects an atomic structure that has a high degree of similarity to the target biomolecule in structure and/or molecular orientation, from among the modified atomic structures, based on a degree of similarity between a second candidate structure image which is included in the plurality of second candidate structure images and the generation image.

*FIG.1*

START

PARTICLE IMAGE IS ACQUIRED — S100

GENERATION IMAGE IS GENERATED — S200

TWO-DIMENSIONAL PROJECTION IMAGES IS ACQUIRED — S300

DEGREE OF SIMILARITY IS CALCULATED — S400

BASIC CANDIDATE STRUCTURES IS SELECTED — S500

GLOBAL STRUCTURE SEARCH

MODIFIED ATOMIC STRUCTURE IS GENERATED — S600

DEGREE OF SIMILARITY IS CALCULATED — S700

IS SEARCH TERMINATED? — S800

No

Yes

LOCAL STRUCTURE SEARCH

THREE-DIMENSIONAL STRUCTURE IS OUTPUT — S900

THREE-DIMENSIONAL STRUCTURE IS EVALUATED — S1000

END

*FIG.2*

FIG.3

FIG.4

STRUCTURE 1          STRUCTURE 2          STRUCTURE 3

T01                  T02                  T03

OVER TIME

*FIG.5*

FIG.6

702

*FIG.7*

*FIG.8*

902

90-DEGREE
ROTATION

904

FIG.9

FIG.10

*FIG.11A*

*FIG.11B*

FIG.12

*FIG.13*

FIG.14A

FIG.14B

FIG.14C

FIG.14D

a.

Target1-LOW

FIG.15A

b.

Target1-VAE

*FIG.15B*

FIG.15C

*FIG.15D*

FIG.15E

*FIG.15F*

*FIG.15G*

FIG.15H

FIG.15I

*FIG.16A*

a.

Target0

e.

PC2

PC1

l.

Similarity-score

RMSD

m.

q.

IFIE heatmap

FIG.16B

FIG.16C

c.

Target3

g.

k.

o.

s.

IFIE heatmap

*FIG.16D*

d.

Target4

h.

PC2

PC1

I.

Similarity-score

RMSD

p.

t.    IFIE heatmap

*FIG.16E*

e.

Target11

k.

q.

m.

q.

IFIE heatmap

*FIG.16F*

**f.** Target13

**l.**

**r.**

**n.**

**r.** IFIE heatmap

*FIG.16G*

*FIG.17A*

FIG.17B

b. Target5

f.

j.

n.

r. IFIE heatmap

FIG.17C

FIG.17D

d. Target25

h.

l.

p.

t. IFIE heatmap

*FIG.17E*

FIG.18A

FIG.18B

FIG.18C

c. Target30

g.

PC2

PC1

k.

MS-score

RMSD

o.

s.   IFIE heatmap

*FIG.18D*

d. Target1

h.

p.

t.     IFIE heatmap

*FIG.18E*

EP 4 773 074 A1

FIG.19

# EP 4 773 074 A1

## INTERNATIONAL SEARCH REPORT

International application No.

**PCT/JP2024/031415**

### A. CLASSIFICATION OF SUBJECT MATTER

*G06T 7/00*(2017.01)i; *G16C 10/00*(2019.01)i; *G16C 20/40*(2019.01)i
FI:    G06T7/00 300D; G06T7/00 300B; G16C10/00; G16C20/40

According to International Patent Classification (IPC) or to both national classification and IPC

### B. FIELDS SEARCHED

Minimum documentation searched (classification system followed by classification symbols)

G06T7/00; G16C10/00; G16C20/40

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2022/092317 A1 (THE UNIVERSITY OF TOKYO) 05 May 2022 (2022-05-05) entire text, all drawings | 1-20 |
| A | EP 3671208 A1 (AARHUS UNIVERSITET) 24 June 2020 (2020-06-24) whole document | 1-20 |
| A | 千葉 峻太朗, 荒木 望嗣, 奥野 恭史, ビッグデータ解析と分子シミュレーション を活用したインシリコ創薬, JOURNAL OF THE JAPAN SOCIETY FOR SIMULATION TECHNOLOGY, 15 March 2018, vol. 37, no. 1, pp. 42-49, (CHIBA, Shuntaro, ARAKI, Mitsugu, OKUNO, Yasushi, Computer-aided Drug Discovery Based on Big Data Analysis and Molecular Simulation) entire text, all drawings | 1-20 |

☐ Further documents are listed in the continuation of Box C.   ☑ See patent family annex.

| Date of the actual completion of the international search | Date of mailing of the international search report |
|---|---|
| **23 October 2024** | **05 November 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

INTERNATIONAL SEARCH REPORT
Information on patent family members

| International application No. |
| --- |
| **PCT/JP2024/031415** |

| Patent document cited in search report | | | Publication date (day/month/year) | Patent family member(s) | Publication date (day/month/year) |
| --- | --- | --- | --- | --- | --- |
| WO | 2022/092317 | A1 | 05 May 2022 | (Family: none) | |
| EP | 3671208 | A1 | 24 June 2020 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHIHONG SONG** ; **KAZUYOSHI MURATA**. *J. Comput. Chem. Jpn.*, 2018, vol. 17 (1), 38-45
- **ELLEN D. ZHONG** ; **TRISTAN BEPLER** ; **BONNIE BERGER** ; **JOSEPH H. DAVIS**. CryoDRGN: reconstruction of heterogeneous cryo-EM structures using neural networks. *Nature Methods*, 2021, vol. 18, 176-185, https://doi.org/10.1038/s41592-020-01049-4
- **ELLEN D. ZHONG** ; **TRISTAN BEPLER** ; **JOSEPH H. DAVIS** ; **BONNIE BERGER**. *Reconstructing continuous distributions of 3D protein structure from cryo-EM images*, https://arxiv.org/abs/1909.05215
- **ALI PUNJANI** ; **DAVID J. FLEET**. 3DFlex: determining structure and motion of flexible proteins from cryo-EM. *Nature Methods*, 2023, vol. 20, 860-870, https://www.nature.com/articles/s41592-023-01853-8